(19) Europäisches Patentamt
European Patent Office
Office européen des brevets

(11) Publication number: **0 181 152**
**B1**

(12) **EUROPEAN PATENT SPECIFICATION**

(45) Date of publication of patent specification: **13.09.89**

(21) Application number: **85307869.9**

(22) Date of filing: **30.10.85**

(51) Int. Cl.[4]: **C 07 D 403/06,**
C 07 D 241/08, A 61 K 31/495
// (C07D403/06, 209:18,
241:08)

(54) **Piperazine compound as PAF-antagonist.**

(30) Priority: **02.11.84 GB 8427735**

(43) Date of publication of application:
**14.05.86 Bulletin 86/20**

(45) Publication of the grant of the patent:
**13.09.89 Bulletin 89/37**

(84) Designated Contracting States:
**AT BE CH DE FR GB IT LI LU NL SE**

(56) References cited:
**EP-A-0 008 186**
**GB-A-1 600 127**
**CHEMICAL ABSTRACTS, vol. 100, no. 25, June 18, 1984, Columbus, Ohio, USA SUMITOMO CHEMICAL CO., LTD. "3,6-Bis(indol-3-ylmethyl)-2,5-piperazinedione" page 603, column 2, abstract-no. 209 873q**

(73) Proprietor: **FUJISAWA PHARMACEUTICAL CO., LTD.**
**3, Doshomachi 4-chome Higashi-ku**
**Osaka-shi Osaka 541 (JP)**

(72) Inventor: **Shimazaki, Norihiko**
**New Life Umezono Daiichi No. 602 No. 2-5-4 Umezono**
**Sakuramura Niihari-gun Ibaraki-ken (JP)**
Inventor: **Hemmi, Keiji**
**No. 2-17-1, Umezono Sakuramura**
**Niihari-gun Ibaraki-ken (JP)**
Inventor: **Nakaguti, Osamu**
**No. 15-34, Nakasakurazuka 1-chome**
**Toyonaka (JP)**
Inventor: **Miyazaki, Yoshio**
**No. 1-13, Hiramatsu 2-chome**
**Itami (JP)**
Inventor: **Hashimoto, Masashi**
**Kashiwa manshion No. 212 No. 2-11-6, Takezono**
**Sakuramura Niihari-gun Ibaraki-ken (JP)**

(74) Representative: **Pennant, Pyers et al**
**Stevens, Hewlett & Perkins 5 Quality Court**
**Chancery Lane**
**London, WC2A 1HZ (GB)**

**EP 0 181 152 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
CHEMICAL ABSTRACTS, vol. 96, no. 25, June 21, 1982, Columbus, Ohio, USA TRAN, Chieu D.; BEDDARD, GODFREY S.; MC CONNEL, ROSE; HOYNG, CHARLES F.; FENDLER, JANOS H. "Ground and excited state conformational differences between diastereomeric dipeptides" page 785, column 2, abstract-no. 218 218f

CHEMICAL ABSTRACTS, vol. 96, no. 25, June 21, 1982, Columbus, Ohio, USA NAKAGAWA, MASAKO; SUGUMI, HIROYUKI; KODATO, SHINICHI; HINO, TOHRU "Oxidative dimerization ofNb-acyltryptophans. Total synthesis and absolute configuration of ditryptophenaline" page 784, column 1, abstract-no. 218 204y

CHEMICAL ABSTRACTS, vol. 86, no. 3, January 17, 1977, Columbus, Ohio, USA HOPE, ALAN P.; HALPERN, BERTHOLD "The application of ketimine derivatives of amino acids to peptide synthesis" page 453, column 1, abstract-no. 16 936w

CHEMICAL ABSTRACTS, vol. 97, no. 9, August 30, 1982, Columbus, Ohio, USA KOZLOVSKY, A.G. "Peculiarities of biosynthesis of alkaloids by fungi" page 317, column 2, abstract-no. 68 957a

CHEMICAL ABSTRACTS, vol. 97, no. 1, July 5, 1982, Columbus,Ohio, USA OTTENHEIJM, HARRY C.J.; PLATE, RALF; NOORDIK, JAN H.; HERSCHEID, JACOBUS D.M. "N-Hydroxytryptophan in the synthesis of natural products containing oxidized dioxopiperazines. An approach to the neoechinulin and sporidesmin series" page 654, column 1, abstract-no. 6 749a

CHEMICAL ABSTRACTS, vol. 90, no. 19, May 7, 1979, Columbus, Ohio, USA OIKAWA, YUJI; YOSHIOKA, TADAO; YONEMITSU, OSAMU "Synthesis of oxidized diketopiperazine alkaloids by the selective oxidation of C-3 side chains of indoles" page 641, column 1, abstract-no. 152 436t

CHEMICAL ABSTRACTS, vol. 94, no. 1, January 5, 1981, Columbus, Ohio, USA GRUNDON, MICHAEL F.; HAMBLIN, MICHAEL R.; HARRISON, DAVID M.; LOGUE, J.N. DERRY; MAGUIRE, MAUREEN; MC GRATH, J. AIDAN "Biosynthesis of aromatic isoprenoids. Part 5. The preparation of 1-(3,3-dimethylallyl)-L-tryptophan and cyclo-L-alanyl-1-(3,3-dimethyl-allyl)-L-tryptophan and their nonincorporation into echinulin" page 399, column 1, abstract-no. 4 235u

CHEMICAL ABSTRACTS, vol. 95, no. 17, October 26, 1981, Columbus, Ohio, USA ARAI, KUNIZO; SATO, SHINGO; SHIMIZU, SAKAE; NITTA, KEIICHI; YAMAMOTO, YUZURU "Metabolic products of Aspergillus terreus. VII. Astechrome: an iron-containg metabolite of the strain IFO 6123" page 663, column 2, abstract-no. 150 589z

(56) References cited:
CHEMICAL ABSTRACTS, vol. 93, no.5, August 4, 1980, Columbus, Ohio, USA OHTA, AKIHIRO; AKITA, YASOU; NAKANE, YUMIKO " Conversion of 2,5-diphenyl- and 2,5-dibenzylpyrazines to 2,5-diketopiperazines" page 926, column 1, abstract-no. 46 584s

CHEMICAL ABSTRACTS, vol.95, no. 1, July 6, 1981, Columbus, Ohio, USA KANMERA, TATSUHIKO; LEE, SANNAMU; AOYAGI, HARUHIKO; IZUMIYA, NOBUO "Cyclic peptides. IX. Asymmetric hydrogenation of alpha,beta-dehydroamino acid residue in cyclodipeptides and preparation of optically pure alpha-amino acids" page 738, column 1, abstract-no. 7 743q

Int. Journal Pept. Protein Res. 1980, 16(4), 280-90

**Description**

This invention relates to new PAF (Platelet Activating Factor) antagonists, piperazine compounds (I) and pharmaceutically acceptable salts thereof. More particularly, it relates to a pharmaceutical composition comprising the piperazine compounds (I) or pharmaceutically acceptable salts thereof as an active ingredient, and to a new piperazine compound (Ia) and pharmaceutically acceptable salts thereof and to processes for the preparation thereof.

This invention is based on the discovery by the present inventors that certain piperazine compounds (I) and pharmaceutically acceptable salts thereof are highly potent antagonists of PAF and are therefore useful for the prevention and treatment of diseases caused by PAF.

Various piperazine derivatives are disclosed in Chemical Abstracts, volume 95, 1, 1981, 7743q; Chemical Abstracts, Volume 96, No. 25, 1982, 218204y; Chemical Abstracts, Volume 100, No. 25, 1984, 209873q and Int. J. Peptide Protein Res. 16, 1980, p. 280—290. In addition, EP—A—0 008 186 and GB—A—1 600 127 describe piperazine-2,5-dione derivatives having a variety of pharmacological properties.

The piperazine compounds used in this invention can be represented by the following formula:

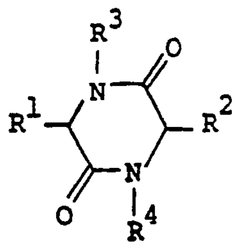

(I)

wherein

$R^1$ is phenyl; mono(or di)phenyl($C_1$—$C_6$)alkyl; naphthyl($C_1$—$C_6$)alkyl; anthryl($C_1$—$C_6$)alkyl; phenyl($C_1$—$C_6$)alkyl; having $C_1$—$C_6$ alkoxy substituent(s); indolyl($C_1$—$C_6$)alkyl; benzothienyl($C_1$—$C_6$)alkyl; pyridyl($C_1$—$C_6$)alkyl; N-$C_1$—$C_6$ alkylindoyl($C_1$—$C_6$)alkyl; or N-$C_1$—$C_6$ alkylindolyl($C_1$—$C_6$)alkyl having phenyl substituent(s) on the indole ring;

$R^2$ is $C_1$—$C_6$ alkyl; $C_7$—$C_{22}$ alkyl; $C_1$—$C_6$ alkylthio($C_1$—$C_6$)alkyl; $C_2$—$C_6$ alkenylthio($C_1$—$C_6$)alkyl; hydroxy($C_1$—$C_6$)alkyl; $C_1$—$C_6$ alkanoyloxy($C_1$—$C_6$)alkyl; phenyl($C_1$—$C_6$)alkoxy($C_1$—$C_6$)alkyl; amino($C_1$—$C_6$)-alkyl; $C_1$—$C_6$ alkanoylamino($C_1$—$C_6$)alkyl; benzamido($C_1$—$C_6$)alkyl; phenyl($C_1$—$C_6$)alkoxycarbonylamino-($C_1$—$C_6$)alkyl; carboxy($C_1$—$C_6$)alkyl; $C_1$—$C_6$ alkoxycarbonyl($C_1$—$C_6$)alkyl; phenyl($C_1$—$C_6$)alkoxy-carbonyl($C_1$—$C_6$)alkyl; phenylthio($C_1$—$C_6$)alkyl; phenyl($C_1$—$C_6$)alkylthio($C_1$—$C_6$)alkyl; or N—$C_1$—$C_6$ alkyl-indolyl($C_1$—$C_6$)alkyl, and

$R^3$ and $R^4$ are each hydrogen or $C_1$—$C_6$ alkyl, or pharmaceutically acceptable salts thereof.

The piperazine compounds (I) include both previously known and novel compounds.

Among the piperazine compounds(I), the new and preferred stereochemically specific compounds are those which can be represented by the following formula:

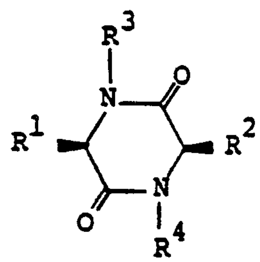

(Ia)

wherein

$R^1$ is phenyl; mono(or di)phenyl($C_1$—$C_6$)alkyl; naphthyl($C_1$—$C_6$)alkyl; anthryl($C_1$—$C_6$)alkyl; phenyl($C_1$—$C_6$)alkyl; having $C_1$—$C_6$ alkoxy substituent(s); indolyl($C_1$—$C_6$)alkyl; benzothienyl($C_1$—$C_6$)alkyl; pyridyl($C_1$—$C_6$)alkyl; N-$C_1$—$C_6$ alkylindolyl($C_1$—$C_6$)alkyl; or N-$C_1$—$C_6$ alkylindolyl($C_1$—$C_6$)alkyl having phenyl substituent(s) on the indole ring;

$R^2$ is $C_1$—$C_6$ alkyl; $C_7$—$C_{22}$ alkyl; $C_1$—$C_6$ alkylthio($C_1$—$C_6$)alkyl; $C_2$—$C_6$ alkenylthio($C_1$—$C_6$)alkyl; hydroxy($C_1$—$C_6$)alkyl; $C_1$—$C_6$ alkanoyloxy($C_1$—$C_6$)alkyl; phenyl($C_1$—$C_6$)alkoxy($C_1$—$C_6$)alkyl; amino($C_1$—$C_6$)-alkyl; $C_1$—$C_6$ alkanoylamino($C_1$—$C_6$)alkyl; benzamido($C_1$—$C_6$)alkyl; phenyl($C_1$—$C_6$)alkoxycarbonyl-amino($C_1$—$C_6$)alkyl; carboxy($C_1$—$C_6$)alkyl; $C_1$—$C_6$ alkoxycarbonyl($C_1$—$C_6$)alkyl; phenyl($C_1$—$C_6$)alkoxy-carbonyl($C_1$—$C_6$)alkyl; phenylthio($C_1$—$C_6$)alkyl; phenyl($C_1$—$C_6$)alkylthio($C_1$—$C_6$)alkyl; or N—$C_1$—$C_6$ alkyl-indolyl($C_1$—$C_6$)alkyl, and

$R^3$ and $R^4$ are each hydrogen or $C_1$—$C_6$ alkyl, or pharmaceutically acceptable salts thereof.

The pharmaceutically acceptable salts of the compound (I) and (Ia) may include organic or inorganic acid addition salts (e.g. hydrochloride) and salts with an organic or inorganic base (e.g. sodium salt).

According to this invention, the piperazine compounds (I) and pharmaceutically acceptable salt thereof can be prepared by, for example, the following processes.

Process 1 :

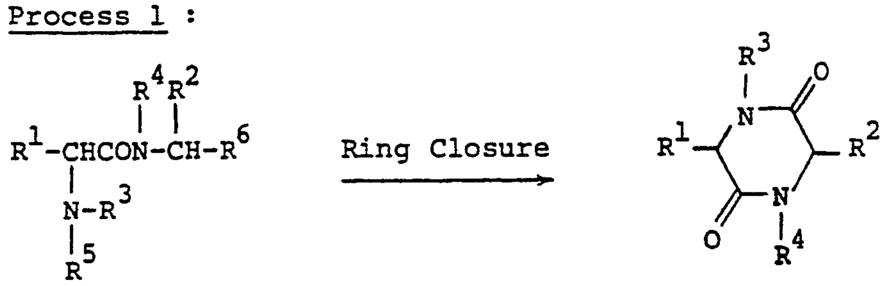


(II) (I)

or its reactive derivative at the carboxy or amino group or salt thereof

or salt thereof

Process 2 :

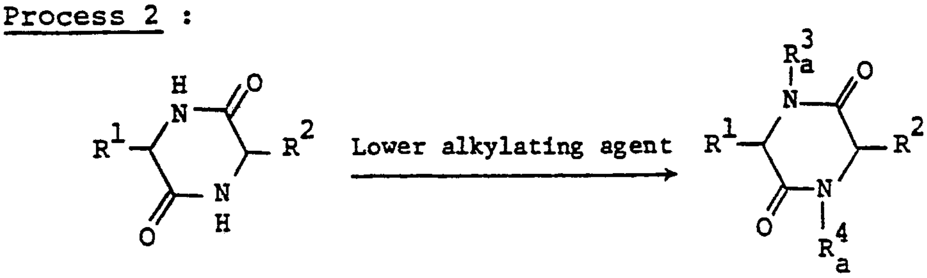


(Ib) (Ic)

or salt thereof

or salt thereof

Process 3 :

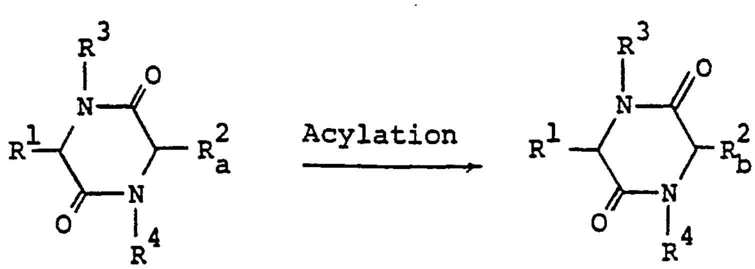


(Id) (Ie)

or its reactive derivative at the amino group or salt thereof

or salt thereof

Process 4 :

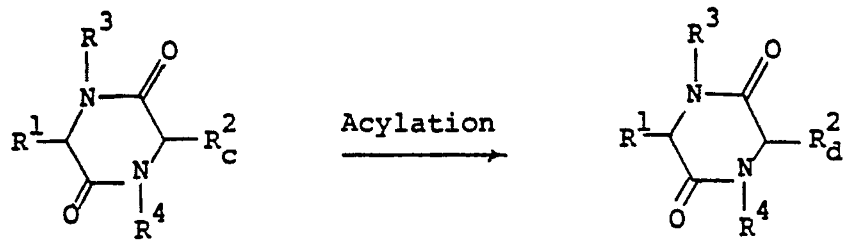


(If) or salt thereof (Ig) or salt thereof

Process 5 :

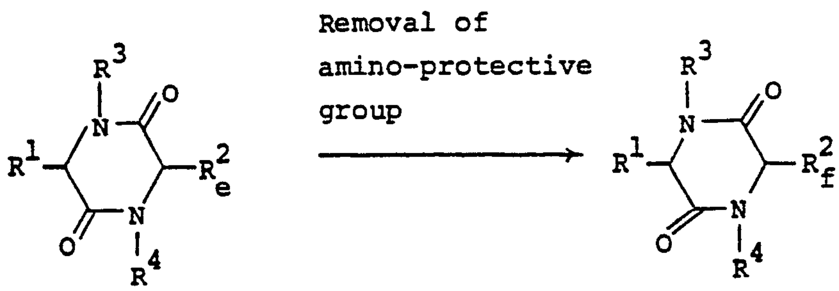


(Ih) or salt thereof (Ii) or salt thereof

Process 6 :

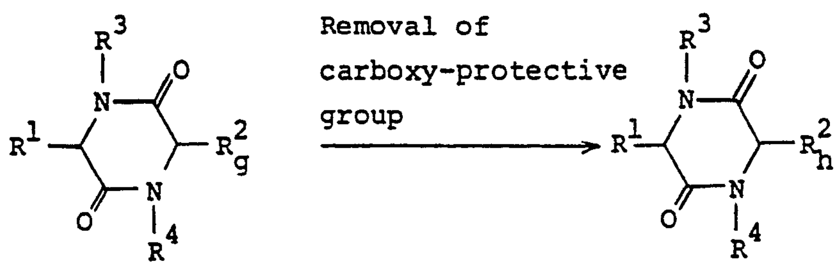


(Ij) or salt thereof (Ik) or salt thereof

wherein

$R^1$, $R^2$, $R^3$ and $R^4$ are each as defined above,
$R^2_a$ and $R^2_f$ are each amino ($C_1$—$C_6$)alkyl,
$R^2_b$ is acylamino($C_1$—$C_6$)alkyl,
$R^2_c$ is hydroxy($C_1$—$C_6$)alkyl,
$R^2_d$ is acyloxy($C_1$—$C_6$)alkyl,
$R^2_e$ is a protected amino($C_1$—$C_6$)alkyl,
$R^2_g$ is a protected carboxy($C_1$—$C_6$)alkyl,
$R^2_h$ is carboxy($C_1$—$C_6$)alkyl,
$R^3_a$ and $R^4_a$ are each $C_1$—$C_6$ alkyl,

$R^5$ is hydrogen or an amino-protective group and

$R^6$ is carboxy or a protected carboxy.

The starting compound (II) includes both previously known compounds and new compounds and can be prepared by subjecting the corresponding two amino acids to peptide linkage formation reaction in a conventional manner.

The definitions used herein for the various substituents will now be explained in more detail.

Suitable "$C_1$—$C_6$ alkoxy" may include methoxy, ethoxy, propoxy, isopropoxy, butoxy, isobutoxy, sec-butoxy, tert-butoxy, pentyloxy and hexyloxy.

Suitable "$C_1$—$C_6$ alkyl" group and "$C_1$—$C_6$ alkyl" moiety in the terms used herein may include methyl, ethyl, propyl, isopropyl, butyl, isobutyl, sec-butyl, tert-butyl, pentyl and hexyl.

Suitable "$C_7$—$C_{22}$ alkyl", which is more preferably $C_7$—$C_{22}$ alkyl, may include heptyl, octyl, decyl, dodecyl, tetradecyl, hexadecyl and octadecyl.

Suitable "$C_2$—$C_6$ alkenyl" moiety in the term "$C_2$—$C_6$ alkenylthio($C_1$—$C_6$)alkyl" may include $C_2$—$C_6$ alkenyl having 2 to 6 carbon atoms such as vinyl, allyl, isopropenyl, 2-butenyl, 3-pentenyl and 4-hexenyl.

The "amino-protective group" and "amino-protective" moiety in the term "protected amino" include a conventional amino-protective group which is used in the field of amino acid and peptide chemistry, and suitable "amino-protective group" may include acyl such as $C_1$—$C_6$ alkanoyl (e.g. formyl, acetyl, propionyl), aroyl (e.g. bezoyl), $C_1$—$C_6$ alkoxycarbonyl (e.g. methoxycarbonyl, ethoxycarbonyl, propoxycarbonyl) and ar($C_1$—$C_6$)alkoxy-carbonyl [e.g. mono(or di or tri)phenyl($C_1$—$C_6$)alkoxycarbonyl, (e.g. benzyloxycarbonyl)].

Suitable "protected carboxy" may include esterified carboxy such as $C_1$—$C_6$ alkoxycarbonyl as aforementioned and ar($C_1$—$C_6$)alkoxycarbonyl as aforementioned.

The salts of the compounds (Ib), (Ic), (Id), (Ie), (If), (Ig), (Ih), (Ii), (Ij), (Ik) and (II) are substantially the same salt as those exemplified in the explanation of the pharmaceutically acceptable salt of the compound (I).

The processes as illustrated above are explained in more detail in the following.

Process 1:

The object compound (I) or salt thereof can be prepared by subjecting the compound (II) or its reactive derivative at the carboxy or amino group or salt thereof to ring closure reaction.

Suitable reactive derivative at the amino group of the compound (II) may include Schiff's base type imino or its tautomeric enamine type isomer formed by the reaction of the compound (II) with a carbonyl compound such as aldehyde, ketone or the like; a silyl derivative formed by the reaction of the compound (II) with a silyl compound such as bis(trimethylsilyl)acetamide, trimethylsilylacetamide or the like.

The suitable reactive derivative at the carboxy of the compound (II) may be a conventional one such as an acid halide (e.g. acid chloride, acid bromide, etc.), an acid azide, an acid anhydride, an activated amide, an activated ester and the like.

When free acid is used as starting compound (II), the reaction may preferably be conducted in the presence of a conventional condensing agent.

The reaction can also be conducted in the presence of an organic or inorganic base such as alkali metal (e.g. sodium), alkaline earth metal (e.g. calcium), alkali or alkaline earth metal hydride (e.g. sodium hydride, calcium hydride, etc.), alkali or alkaline earth metal hydroxide (e.g. sodium hydroxide, potassium hydroxide, calcium hydroxide, etc.), alkali or alkaline earth metal carbonate or bicarbonate (e.g. sodium carbonate, potassium carbonate, sodium bicarbonate), alkali or alkaline earth metal alkoxide (e.g. sodium ethoxide, lithium methoxide, magnesium methoxide), trialkylamine (e.g. triethylamine), pyridine, bicyclodiaza compound (e.g. 1,5-diazabicyclo[3,4,0]nonene-5, 1,5-diazabicyclo[5,4,0]undecene-5, etc.) and the like.

This reaction is usually carried out without solvent or in the presence of a solvent which does not adversely influence the reaction such as methanol, ethanol, propanol, acetic acid, dimethylformamide, dichloroethane, toluene, benzene, xylene and the like.

The reaction temperature is not critical, and the reaction is usually carried out under heating to under cooling.

When the compound (II) having amino-protective group for $R^5$ is used as a starting compound, it may often give preferred results to remove the amino-protective group in a conventional manner (e.g. hydrolysis, catalytic reduction, etc.) prior to this ring closure reaction.

Process 2:

The object compound (Ic) or salts thereof can be prepared by reacting the compound (Ib) or salt thereof with a lower alkylating agent.

The lower alkylating agent may include lower alkyl halide (e.g. methyl iodide, etc.), di(lower)alkylsulfate (e.g. dimethylsulfate, etc.), diazo(lower)alkane (e.g. diazomethane, etc.), lower alkyl sulfonate (e.g. methyl sulfonate, etc.) and the like.

This reaction can be conducted in the presence of an organic or inorganic base as those exemplified in the explanation of Process 1.

This reaction is usually carried out in the presence of a solvent which does not adversely influence the reaction such as dichloromethane, dimethylformamide and the like.

The reaction temperature is not critical and the reaction is sufficiently carried out at ambient temperature.

Process 3:

The object compound (Ie) or salt thereof can be prepared by reacting the compound (Id) or its reactive derivative at the amino group or salt thereof with an acylating agent.

Suitable reactive derivative at the amino group of the compound (Id) is substantially the same as that of the compound (II) explained in the Process 1.

The acylating agent to be used in this reaction includes an organic acid (i.e. $R^7$ OH (IV), in which $R^7$ is acyl) and its reactive derivative.

The suitable reactive derivative of the compound (IV) is substantially the same as that of the compound (II) explained in Process 1.

When free acid is used as an acylating agent, the acylation reaction may preferably be conducted in the presence of a conventional condensing agent.

The reaction can preferably be conducted in the presence of an organic or inorganic base as those exemplified in the explanation of the above Process 1.

This reaction is usually carried out in a solvent which does not adversely influence the reaction such as methanol, ethanol, propanol, tetrahydrofuran, chloroform, pyridine and the like

The reaction temperature is not critical and the reaction can be carried out under heating to under cooling.

Process 4:

The compound (Ig) or salt thereof can be prepared by reacting the compound (If) or salt thereof with an acylating agent.

The reaction conditions and the acylating agent are substantially the same as those explained in the Process 3.

Process 5:

The compound (Ii) or salt thereof can be prepared by subjecting the compound (Ih) or salt thereof to removal reaction of amino-protective group.

The removal reaction of this process is carried out in a conventional manner (e.g. hydrolysis, catalytic reduction, reduction, etc.).

The hydrolysis is preferably carried out in the presence of inorganic or organic acid (e.g. hydrochloric acid, sulfuric acid, acetic acid, trifluoroacetic acid, etc.), or inorganic or organic base (e.g. sodium hydroxide, etc.).

The catalytic reduction can be carried out in the presence of a conventional catalyst for catalytic reduction (e.g. paradium on charcoal, etc.) at atmospheric pressure.

The reaction of this process is usually carried out in a solvent which does not adversely influence the reaction such as methanol, ethanol, propanol, acetic acid and the like, at a temperature range of cooling to heating.

Process 6:

The compound (Ik) or salt thereof can be prepared by subjecting the compound (Ij) or salt thereof to removal reaction of the carboxy-protective group.

The removal reaction is carried out in substantially the same manner as that of Process 5.

The object compound (I), (Ic), (Ie), (Ig), (Ii) and (Ik) in the above processes can be purified and converted to the desired salts in a conventional manner.

The object compounds (I) and pharmaceutically acceptable salt thereof are antagonists of PAF and therefore useful as a medicine for preventing and treating diseases caused by PAF such as allergic manifestation (e.g. asthma, etc.), thrombosis, nephritis, endotoxin shock (ARDS), and the like.

The following Tests are given for the purpose of illustrating antagonism of the compounds (I) against PAF.

The test compounds are listed below.

Test compound

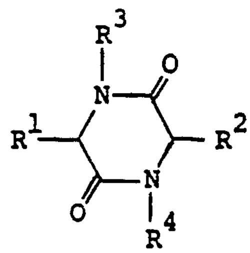

| Test compound No. | $R^1$ | $R^2$ | $R^3$ | $R^4$ |
|---|---|---|---|---|
| 1 | 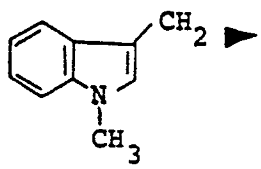 | $CH_2CH_2-SCH_3$ | H | H |
| 2 | 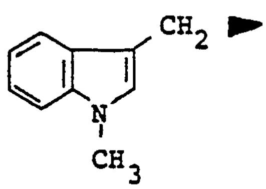 | 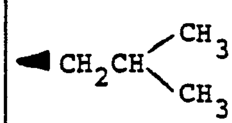 | H | H |

| Test compound No. | $R^1$ | $R^2$ | $R^3$ | $R^4$ |
|---|---|---|---|---|
| 3 | 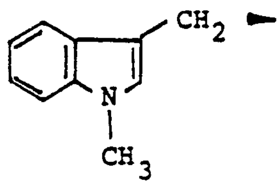 | ◄ $CH_2$-$SCH_3$ | H | H |
| 4 | 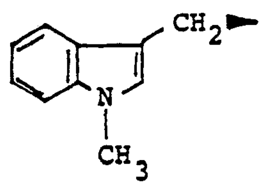 | ◄ $CH_2CH_2SCH_3$ | $-CH_3$ | $-CH_3$ |
| 5 | 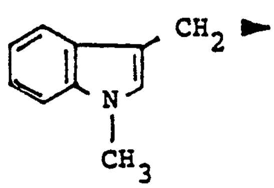 | ∿ $(CH_2)_3SCH_3$ | H | H |
| 6 | 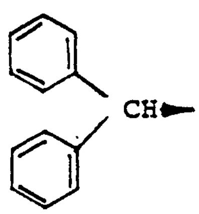 | ◄ $CH_2CH_2SCH_3$ | H | H |
| 7 | 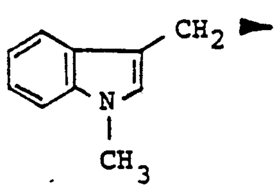 | 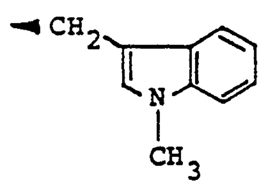 | H | H |

| Test compound No. | $R^1$ | $R^2$ | $R^3$ | $R^4$ |
|---|---|---|---|---|
| 8 | 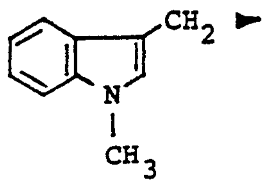 | $(CH_2)_3CH_3$ | H | H |
| 9 | 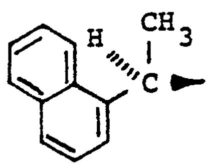 | $CH_2CH_2SCH_3$ | H | H |
| 10 | 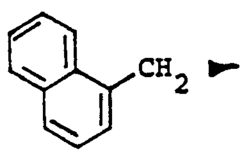 | $CH_2CH_2SCH_3$ | H | H |
| 11 | 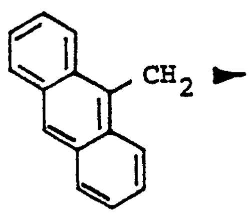 | $CH_2CH_2SCH_3$ | H | H |
| 12 | 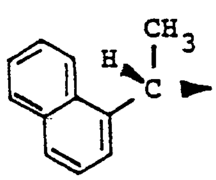 | $CH_2CH_2SCH_3$ | H | H |

| Test compound No. | $R^1$ | $R^2$ | $R^3$ | $R^4$ |
|---|---|---|---|---|
| 13 | 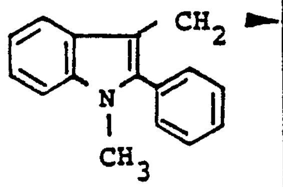 | $CH_2CH_2SCH_3$ | H | H |
| 14 | 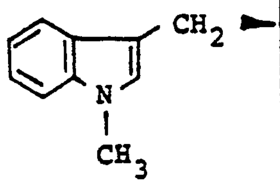 | $CH_2CH_2CH_3$ | H | H |
| 15 | 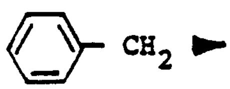 | 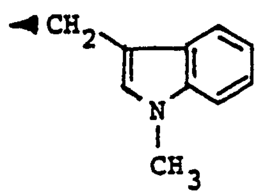 | H | H |
| 16 | 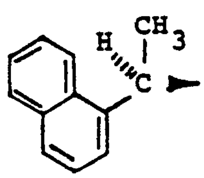 | $CH_2CH_2SCH_3$ | $-CH_3$ | $-CH_3$ |
| 17 | 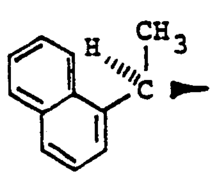 | $(CH_2)_3CH_3$ | $-CH_3$ | $-CH_3$ |

Test 1 (inhibition of platelet aggregation)

Test method

Blood was collected through the polyethylene catheter introduced into the carotid artery of male Japanese white rabbit (2.5 to 3 kg body weight). The blood was anticoagulated with 1 volume of 3.8% sodium citrate to 9 volume of blood. Platelet rich plasma (PRP) was prepared by centrifugation of the blood at 150 g for 10 minutes at room teperature. The PRP was diluted with platelet poor plasma obtained by further centrifugation of the blood at 1,000 g for 20 minutes. The platelet number was $5 \times 10^5$ cells/$mm^3$. Platelet aggregation induced with PAF was measured by the nephelometric technique of Born and Cross (cf. Journal of Physiology *168*, 178—188 (1963) using HKK Hema tracer (trade name, made by Niko Bioscience Inc.).

Activities of inhibitors were expressed at $IC_{50}$ value, i.e. concentrations required to inhibit the platelet aggregation response by 50%. The final concentration of PAF was usually 20 nM. The test results are shown in the following table.

Test result

| Test compound No. | $IC_{50}$ (μg/mℓ) |
|---|---|
| 1 | 0.71 |
| 2 | 1.04 |
| 3 | 2.5 |
| 4 | 0.84 |
| 5 | 0.55 |
| 6 | 0.75 |
| 7 | 1.2 |
| 8 | 0.58 |
| 9 | 0.061 |
| 10 | 0.48 |
| 11 | 0.58 |
| 12 | 0.65 |
| 13 | 0.72 |
| 14 | 0.93 |
| 15 | 0.82 |
| 16 | 0.59 |
| 17 | 0.55 |

Test 2 (Inhibition of hypotensive effect induced by intraveneously injected PAF in rats)

Test method

Seven-week old Sprague-Dowley rats were anesthetized with urethane (1 g/kg, i.p.). Catheters were introduced into the femoral artery and vein for the measurement of arterial pressure and the administration of the test compound, respectively. Blood pressure was recorded from femoral artery using a transducer coupled to the Biophysiograph 180 System (San-Ei Instrument Co., Ltd.). Inhibitory activity of the test compound was shown in the following Table as the inhibition percentage of the syntehtic PAF induced hypotension. PAF was administered intravenously at a dose of 1 µg/kg. The test compound was administered intravenously 3 minutes before PAF injection.

Test result

| Test compound No. | Dose (mg/kg) | Inhibition maximum(%) of hypotensive effect |
|---|---|---|
| 2 | 10 | 75 |
| 9 | 3 | 51 |

Test 3 (inhibition of vascular premeability increase induced by intradermally injected PAF in mice)

Test method

Six week old male ddY mice were used. The test compound was administered intraperitoneally 15 minutes before the Evans blue injection (0.125% solution in saline, 0.2 ml/mouse, i.v.).

PAF (50 ng, 50 µl per site) was injected intradermally to the depilated back of the mice 5 minutes after the Evans blue injection.

The inhibitory activity of the test compound was monitored 30 minutes after the PAF injection by assessment of blueing reaction with Evans blue dye in mice skin.

The test results are shown in the following table.

| Test compound No. | Dose (mg/kg) | Inhibition of vascular permeability increase (%) |
|---|---|---|
| 2 | 10 | 30 |
| 9 | 10 | 40 |

The compounds (I) or pharmaceutically acceptable salt thereof in admixture with pharmaceutically acceptable carriers can be administered orally or parenterally to mammals including human being in a form of a pharmaceutical composition such as capsules, tablets, granules, powders, buccal tablets, sublingual tablets, and solutions.

The pharmaceutically acceptable carriers may include various organic or inorganic carrier materials, which are conventionally used for pharmaceutical purpose, such as excipient (e.g. sucrose, starch, mannit, sorbit, lactose, glucose, cellulose, talc, calcium phosphate, calcium carbonate, etc.), binding agent (cellulose, methyl cellulose, hydroxypropylcellulose, polypropylpyrrolidone, gelatin, gum arabic, polyethyleneglycol, sucrose, starch, etc.). disintegrator (e.g. starch, carboxymethyl cellulose, calcium salt of carboxymethyl cellulose, hydroxypropyl-starch, sodium glycole-starch, sodium bicarbonate, calcium phosphate, calcium citrate, etc.), lubricant (e.g. magnesium stearate, aerosil, talc, sodium laurylsulfate, etc.), flavoring agent (e.g. citric acid, mentol, ammonium salt of glycyrrhizin, glycine, orange powders, etc.), preservative (sodium benzoate, sodium bisulfite, methylparaben, propylparaben, etc.), stabilizer (citric acid, sodium citrate, acetic acid, etc.), suspending agent (e.g. methyl cellulose, polyvinylpyrrolidone,

aluminum stearate, etc.), dispersing agent [e.g. surface active agent, etc.], aqueous diluting agent (e.g. water), base wax (e.g. cacao butter, polyethyleneglycol, white petrolatum, etc.).

A dosage of the object compound is to be varied depending on various factors sich as kind of diseases, weight and/or of a patient, and further the kind of administration route.

The optimal dosage of the compounds (I) is usually selected from a dose range of 1 mg—1 g/day, preferably 10 mg—500 mg/day.

The total daily amount mentioned above may be divisionally given to the patient at the interval of 6—12 hours per day.

The following Examples are given for the purpose of illustrating this invention.

Example 1

(1) To a suspension of N-tert-butoxycarbonyl-1-methyl-D-tryptophan (1.60 g) and hydrochloric acid salt of D-methionine ethyl ester (1.06 g) in dry methylene chloride (30 ml) which was cooled on an ice bath and stirred was added a solution of triethylamine (0.50 g) in dry methylene chloride (10 ml). The mixture was stirred for 30 minutes, and to the mixture was added a solution of N,N'-dichlorohexylcarbodiimide (1.04 g) in dry methylene chloride (10 ml). The resulting mixture was stirred overnight. After removal of the solvent, crude residue was taken up with a mixture of ethyl acetate and water, filtered, and organic layer was washed with 10% hydrochloric acid and saturated sodium bicarbonate solution, dried over magnesium sulfate, and evaporated to give crystalline N-tert-butoxycarbonyl-1-methyl-D-tryptophyl-D-methionine ethyl ester (2.43 g).

NMR ($CDCl_3$) δ: 1.21 (3H, t, J=7Hz), 1.36 (9H, s), 1.95 (2H, m), 1.97 (3H, s), 2.25 (2H, m), 3.13 (1H, dd, J=14Hz, 7Hz), 3.35 (1H, dd, J=14Hz, 6Hz), 3.73 (3H, s), 4.14 (2H, q, J=7Hz), 4.50 (2H, m), 5.12 (1H, d, J=7Hz), 6.50 (1H, d, J=7Hz), 6.95 (1H, s), 7.25 (3H, m), 7.66 (1H, m).

(2) A mixture of N-tert-butoxycarbonyl-1-methyl-D-tryptophyl-D-methionine ethyl ester (2.4 g), formic acid (25 ml), and saturated solution of hydrogen chloride in formic acid (10 ml) was stirred on an ice bath for 1.5 hours. After removal of the solvent, the crude residue was dissolved in ethanol (20 ml) and saturated ammonia in ethanol (10 ml) was added. The resulting mixture was stirred for 16 hours at room temperature and precipitated mass was collected, washed with ethanol, and dried. Recrystallization from ethanol gave (3R,6R)-3-(1-methylindol-3-ylmethyl)-6-(2-methylthioethyl)piperazine-2,5-dione (1.16 g).

mp: 229—231°C.

IR (Nujol): 1165 $cm^{-1}$.

NMR (DMSO-$d_6$) δ: 1.05 (2H, m), 1.50 (2H, m), 1.65 (3H, s), 2.90 (1H, dd, J=14Hz, 5Hz), 3.25 (1H, dd, J=14Hz, 5Hz), 3.62 (1H, m), 3.67 (3H, s), 4.08 (1H, m), 6.98 (1H, s), 7.00 (2H, m), 7.30 (1H, dd, J=7Hz, 2Hz), 7.59 (1H, dd, J=7Hz, 2Hz), 7.91 (1H, m), 8.08 (1H, m).

Example 2

A mixture of N-tert-butoxycarbonyl-D-phenylalanyl-S-methyl-D-cysteine ethyl ester (0.41 g), ethyl acetate (10 ml), and saturated solution of hydrogen chloride in ethyl acetate (10 ml) was stirred for 4 hours at room temperature. After removal of the solvent, the residue was dissolved in ethanol (10 ml) and saturated ammonia in ethanol (10 ml) was added to the solution. The resulting mixture was stirred for 17 hours at room temperature and the solvent was evaporated off. Recrystallization from acetic acid gave (3R,6S)-3-benzyl-6-methylthiomethylpiperazine-2,5-dione (0.08 g).

mp: 246—247°C.

IR (Nujol): 1660 $cm^{-1}$.

NMR (DMSO-$d_6$) δ: 1.63 (1H, dd, J=14Hz, 7Hz), 1.85 (3H, s), 2.35 (1H, dd, J=14Hz, 4Hz), 2.92 (1H, dd, J=14Hz, 5Hz), 3.18 (1H, dd, J=14Hz, 5Hz), 3.82 (1H, m), 4.21 (1H, m), 7.22 (5H, s), 7.95 (1H, bs), 8.18 (1H, bs).

Example 3

The following compounds were prepared in a similar manner to those of Example 1 and 2.

(1) (3R,6R)-3-Benzyl-6-(2-methylthioethyl)piperazine-2,5-dione

mp: 253—254°C.

IR (Nujol): 1665 $cm^{-1}$.

NMR (DMSO-$d_6$) δ: 1.15 (2H, m), 1.90 (3H, s), 1.95 (2H, m), 2.88 (1H, dd, J=13Hz, 5Hz), 3.20 (1H, dd, J=13Hz, 3Hz), 3.75 (1H, m), 4.23 (1H, m), 7.25 (5H, m), 8.10 (1H, s), 8.23 (1H, s).

(2) (3S,6R)-3-Benzylthiomethyl-6-(1-methylindol-3-ylmethyl)piperazine-2,5-dione.

mp: 229—231°C.

IR (Nujol): 3180, 3050, 1660 $cm^{-1}$.

NMR (DMSO-$d_6$) δ: 1.73 (1H, dd, J=14Hz, 7Hz), 2.35 (1H, dd, J=14Hz, 4Hz), 3.16 (2H, m), 3.40 (2H, s), 3.65 (3H, s), 3.85 (1H, m), 4.13 (1H, m), 6.85—7.6 (10H, m), 7.92 (1H, d, J=1Hz), 8.06 (1H, d, J=0.5Hz).

(3) (3R,6S)-3-Benzyl-6-methylthiomethylpiperazine-2,5-dione.
mp: 246—247°C.
IR (Nujol): 1660 $cm^{-1}$.
NMR ($DMSO-d_6$) δ: 1.63 (1H, dd, J=14Hz, 7Hz), 1.85 (3H, s), 2.35 (1H, dd, J=14Hz, 4Hz), 2.92 (1H, dd, J=14Hz, 5Hz), 3.18 (1H, dd, J=14Hz, 5Hz), 3.82 (1H, m), 4.21 (1H, m), 7.22 (5H, s), 7.95 (1H, bs), 8.13 (1H, bs).

(4) (3R,6R)-3-Indol-3-ylmethyl-6-(2-methylthioethyl)piperazine-2,5-dione.
mp: 240—241°C.
IR (Nujol): 3350, 3210, 3070, 1670 $cm^{-1}$.
NMR ($DMSO-d_6$) δ: 1.20 (2H, m), 1.70 (2H, m), 1.76 (3H, s), 3.18 (2H, m), 3.72 (1H, m), 4.19 (1H, m), 7.00 (3H, m), 7.20 (1H, m), 7.55 (1H, m), 7.90 (1H, m), 8.05 (1H, m), 10.68 (1H, bs).

(5) (3R,6S)-3-Indol-3-ylmethyl-6-methylthiomethylpiperazine-2,5-dione.
mp: 265—268°C (dec.).
IR (Nujol): 3430, 3190, 3050, 1680, 1660 $cm^{-1}$.
NMR ($DMSO-d_6$) δ: 1.58 (1H, dd, J=14Hz, 7Hz), 1.70 (3H, s), 2.30 (1H, dd, J=14Hz, 4Hz), 3.06 (1H, dd, J=17Hz, 6Hz), 3.35 (1H, dd, J=17Hz, 6Hz), 3.80 (1H, m), 4.16 (1H, m), 7.05 (3H, m), 7.35 (1H, dd, J=6Hz, 2Hz), 7.58 (1H, dd, J=6Hz, 2Hz), 7.90 (1H, bs), 8.08 (1H, bs), 10.85 (1H, bs).

(6) (3R,6RS)-3-Methylthiomethyl-6-phenethylpiperazine-2,5-dione.
mp: 221—228°C.
IR (Nujol):3310, 3180, 3030 1685 $cm^{-1}$.
NMR ($DMSO-d_6$) δ: 2.10 (2H, m), 2.11 (3H, s), 2.70 (2H, m), 2.90 (2H, m), 4.00 (1H, m), 4.20 (1H, m), 7.25 (5H, m), 8.13 (1H, bs), 8.40 (1H, bs).

(7) (3S,6R)-3-Allylthiomethyl-6-(1-methylindol-3-ylmethyl)piperazine-2,5-dione.
mp: 210—211°C.
IR (Nujol): 3180, 1660 $cm^{-1}$.
NMR ($DMSO-d_6$) δ: 1.78 (1H, dd, J=14,6Hz), 2.35 (1H, dd, J=14,4Hz), 2.85 (2H, d, J=7Hz), 3.19 (2H, m), 3.70 (3H, s), 3.80 (1H, m), 4.13 (1H, m), 4.92 (1H, bs), 5.07 (1H, m), 5.60 (1H, m), 7.04 (2H, m), 7.07 (1H, s), 7.36 (1H, d, J=7Hz), 7.56 (1H, d, J=7Hz), 7.92 (1H, bs), 8.06 (1H, bs).

(8) (3R,6S)-3-(Indol-3-ylmethyl)-6-(2-methylthioethyl)piperazine-2,5-dione.
mp: 254—255°C.
IR (Nujol): 3430, 3200, 1680 $cm^{-1}$.
NMR ($DMSO-d_6$) δ: 1.80 (2H, m), 1.95 (3H, s), 2.40 (2H, m), 3.15 (2H, m), 3.35 (1H, m), 4.15 (1H, m), 7.10 (3H, m), 7.40 (1H, m), 7.63 (1H, m), 7.97 (1H, s), 8.11 (1H, d, J=0.5Hz), 10.84 (1H, bs).

(9) (3R,6R)-3-n-Butylthiomethyl-6-(1-methylindol-3-ylmethyl)piperazine-2,5-dione.
mp: 200—201°C.
IR (Nujol): 3180 1675 $cm^{-1}$.
NMR ($DMSO-d_6$) δ: 0.84 (3H, m), 3.35 (4H, m), 2.4 (2H, m), 2.65 (1H, dd, J=14,4Hz), 2.87 (1H, dd, J=14,4Hz), 3.16 (2H, m), 3.47 (1H, m), 3.71 (3H, s), 4.20 (1H, m), 7.10 (3H, m), 7.40 (1H, m), 7.65 (1H, m), 7.85 (1H, bs), 8.18 (1H, bs).

(10) (3R,6S)-3-(1-Methylindol-3-ylmethyl)-6-(2-methylthioethyl)piperazine-2,5-dione.
mp: 234—235°C.
IR (Nujol): 3190, 3050, 1675 $cm^{-1}$.
NMR ($DMSO-d_6$) δ: 1.80 (2H, m), 1.94 (3H, s), 2.35 (2H, m), 3.20 (3H, m), 4.70 (3H, s), 5.07 (1H, m), 6.95 (1H, m), 7.04 (1H, s), 7.15 (1H, m), 7.36 (1H, d, J=7.5Hz), 7.59 (1H, d, J=7.5Hz), 7.95 (1H, s), 8.05 (1H, bs).

(11) (3R,6R)-3-(Benzo[b]thien-3-ylmethyl)-6-(2-methylthioethyl)piperazine-2,5-dione.
IR (Nujol): 3180, 1660 $cm^{-1}$.
NMR ($DMSO-d_6$) δ: 1.25 (2H, m), 1.78 (2H, m), 1.80 (3H, s), 3.20 (1H, dd, J=14,5Hz), 3.43 (1H, dd, J=14,4Hz), 3.76 (1H, m), 4.30 (1H, m), 7.35 (2H, m), 7.40 (1H, s), 7.95 (2H, m), 8.10 (1H, bs), 8.26 (1H, bs).

(12) (3RS,6S)-3-Diphenylmethyl-6-methylthiomethylpiperazine-2,5-dione.
mp: 250—253°C.
IR (Nujol): 3180, 3050, 1670, 1655 $cm^{-1}$.

(13) (3S,6RS)-3-Methylthiomethyl-6-((RS)-1-phenylethyl)piperazine-2,5-dione.
mp: 258—261°C.
IR (Nujol): 3180, 3050, 1655 $cm^{-1}$.

(14) (3R,6RS)-3-Methylthiomethyl-6-((RS)-1-phenylethyl)piperazine-2,5-dione.

mp: 264—264.5°C.

IR (Nujol): 3190, 3050, 1660 $cm^{-1}$.

NMR (DMSO-$d_6$) δ: 0.82 (0.5H, d, J=9Hz), 0.97 (0.5H, d, J=9Hz), 1.34 (3H, d, J=7.5Hz), 1.80 (1.5H, s), 1.96 (1.5H, s), 2.06 (0.5H, d, J=4Hz), 2.21 (0.5H, d, J=4Hz), 2.70 (1H, m), 3.50 (1H, m), 4.00 (1H, m), 7.20 (5H, m), 7.79 (0.5H, s), 7.89 (0.5H, d, J=3Hz), 8.43 (1H, m).

(15) (3RS,6R)-6-Methylthiomethyl-6-phenylpiperazine-2,5-dione.

mp: 189—190°C.

IR (Nujol): 3200, 1675 $cm^{-1}$.

NMR (DMSO-$d_6$) δ: 2.07 and 2.13 (3 × 0.25 H, s, 3 × 0.75H, s, respectively), 2.85—3.20 (2H, m), 4.25 and 4.37 (2 × 0.25H, m, 2 × 0.75H, m, respectively), 5.00 and 5.02 (1 × 0.25H, s, 1 × 0.75H, s, respectively), 7.40 (5H, m), 8.16 and 8.25 (1 × 0.75H, bs, 1 × 0.25H, bs, respectively), 8.67 (1H, bs).

(16) (3R,6R)-3-Isobutyl-6-(1-methylindol-3-ylmethyl)piperazine-2,5-dione.

mp: 234—235°C.

IR (Nujol): 3200, 1665 $cm^{-1}$.

NMR (DMSO-$d_6$) δ: 0.15 (1H, m), 0.45 (3H, d, J=8Hz), 0.51 (3H, d, J=8Hz), 0.75 (1H, m), 1.20 (1H, m), 3.00 (1H, dd, J=14Hz, 5Hz), 3.26 (1H, m), 3.45 (1H, m), 3.70 (3H, s), 4.10 (1H, m), 6.96 (1H, m), 7.02 (1H, s), 7.10 (1H, m), 7.35 (1H, d, J=8Hz), 7.60 (1H, d, J=8Hz), 7.92 (1H, m), 8.03 (1H, m).

Example 4

To a solution of (3R,6R)-3-(1-methylindol-3-ylmethyl)-6-(2-methylthioethyl)piperazine-2,5-dione (0.33 g) in dry dimethylformamide (20 ml) was added potassium tert-butoxide (0.27 g). After stirring at room temperature for 30 minutes, methyl iodide (0.5 ml) was added to the mixture. The reaction mixture was stirred for 5 minutes, diluted with water (200 ml), acidified to pH 3 with 10% hydrochloric acid, and extracted with ethyl acetate (50 ml × 2). Combined organic extract was washed with water, dried over magnesium sulfate, and evaporated in vacuo. The residue (0.25 g) was chromatographed on silica gel (11 g) using chloroform as eluent to give (3R,6R)-1,4-dimethyl-3-(1-methylindol-3-ylmethyl)-6-(2-methylthioethyl)piperazine-2,5-dione (0.08 g) as an oil.

IR ($CHCl_3$): 2990, 2920, 1650 $cm^{-1}$.

NMR ($CDCl_3$) δ: 0.40 (1H, m), 0.75 (1H, m), 1.83 (3H, s), 2.06 (2H, dd, J=7Hz, 7Hz), 2.70 (3H, s), 3.00 (3H, s), 3.23 (1H, dd, J=14Hz, 4Hz), 3.54 (1H, dd, J=6Hz, 4Hz), 3.70 (1H, dd, J=14Hz, 4Hz), 3.73 (3H, s), 4.19 (1H, t, J=4Hz), 6.80 (1H, s), 7.20 (3H, m), 7.55 (1H, m).

Example 5

(1) Sodium borohydride (2.1 g) was added to a solution of 1-acetylnaphthalene (18.86 g) in ethanol (200 ml) at room temperature and the mixture was stirred at the same temperature for 2 hours. The mixture was diluted with water (50 ml), acidified to pH 3 with 1N hydrochloric acid and concentrated to about 70 ml. The concentrate was extracted with ethyl acetate (100 ml × 2). The extract was dried over magnesium sulfate and evaporated to give 1-(1-hydroxyethyl)naphthalene as an oil which was used in a next reaction without further purification. Hydrogen bromide was bubbled into a solution of 1-(1-hydroxyethyl)naphthalene (18.96 g) in ether (180 ml) for 2 hours under ice-bath cooling. The reaction mixture was washed with water (50 ml × 2), 2.5% sodium bicarbonate (50 ml × 2) and water (50 ml × 2), dried over magnesium sulfate and evaporated to give 1-(1-bromoethyl)naphthalene (22.63 g).

NMR ($CDCl_3$) δ: 2.23 (3H, d, J=6Hz), 5.97 (1H, q, J=6Hz), 7.2—8.3 (7H, m).

(2) 2-Acetylaminopropanedioic acid diethyl ester (20.8 g) was added to a solution ethoxide in ethanol (prepared from sodium (2.2 g) and ethanol (100 ml)). After the mixture was stirred for 10 minutes at ambient temperature, 1-(1-bromoethyl)naphthalene was added. The reaction mixture was stirred for 18 hours at room temperature. The precipitated solid was collected by filtration and dissolved in chloroform. The chloroform solution was dried over magnesium sulfate and evaporated to give 2-acetylamino-1-[1-(1-naphthyl)ethyl]propanedioic acid diethyl ester (21.59 g).

NMR ($CDCl_3$) δ: 0.87 (3H, t, J=9Hz), 1.27 (3H, t, J=9Hz), 1.58 (3H, d, J=9Hz), 1.97 (3H, s), 3.1—4.0 (2H, m), 4.27 (2H, q, J=2Hz), 5.00 (1H, q, J=9Hz), 6.57 (1H, s), 7.2—8.3 (7H, m).

(3) A solution of 2-acetylamino-2-[1-(1-naphthyl)ethyl]propanedioic acid diethyl ester (21.59 g) in a mixture of concentrated hydrochloric acid (100 ml) and acetic acid (70 ml) was refluxed for 13 hours. The mixture was concentrated under reduced pressure and the residue was dissolved in water (100 ml). The solution was neutralized to pH 5 with 30% sodium hydroxide. The precipitated crystalline solid was collected by filtration to give 2-amino-3-(1-naphthyl)butyric acid (9.76 g).

Mass: 229 ($M^+$).

(4) A solution of di-tert-butyl dicarbonate (13.95 g) in dioxane (128 ml) was added to a solution of 2-amino-3-(1-naphthyl)butyric acid (9.76 g) in 1N sodium hydroxide (128 ml) and the mixture was stirred for 1

hour at 50°C. The mixture was acidified to pH 3 with concentrated hydrochloric acid and concentrated to about 100 ml. The concentrate was extracted with ethyl acetate (100 ml × 2). The extract was dried over magnesium sulfate and evaporated. The residue was chromatographed on a silica gel (500 ml) using chloroform-methanol (50:1) as eluent to give erythro-2-tert-butoxycarbonylamino-3-(1-naphtyl)butyric acid (6.58 g).

NMR ($CDCl_3$) δ: 1.3—1.6 (12H, br s), 3.9—5.0 (3H, m), 7.3—8.3 (8H, m).

Mass: 3.29 ($M^+$).

(5) To a suspension of erythro-2-tert-butoxycarbonylamino-3-(1-naphthyl)butyric acid (2.50 g) and hydrochloric acid salt of D-methionine ethyl ester (1.62 g) in dry methylene chloride (60 ml) which was cooled on an ice bath and stirred was added triethylamine (0.77 g). The mixture was stirred at ambient temperature for 30 minutes, and to the mixture was added N,N'-dicyclohexylcarbodiimide (1.57 g). The resulting mixture was stirred overnight. After removal of the solvent, crude residue was taken up with a mixture of ethyl acetate and water and filtered and the organic layer was washed with 10% hydrochloric acid and saturated aqueous solution of sodium bicarbonate, dried over magnesium sulfate and evaporated to give a solid (4.29 g). To a solution of the solid in ethyl acetate (40 ml) was added a saturated solution of hydrogen chloride in ethyl acetate (40 ml) and the mixture was set aside at room temperature overnight. After removal of the solvent, crude residue was chromatographed on silica gel (200 g, a mixture of chloroform and methanol as an eluent).

The fractions containing the object compound was collected and concentrated under reduced pressure to give hydrochloric acid salt of N-[(2R,3R)-2-amino-3-(1-naphthyl)butyryl]-D-methionine ethyl ester (1.60 g) as an oil.

IR (Neat): 3200, 1710, 1670 $cm^{-1}$.

(6) To a solution of hydrochloric acid salt of N-[(2R,3R)-2-amino-3-(1-naphthyl)butyryl]-D-methionine ethyl ester (1.60 g) in ethanol (30 ml) was added a saturated solution of ammonia in ethanol (30 ml) and the mixture was set aside overnight at ambient temperature, and precipitated mass was collected, washed with ethanol, and dried to give (3R,6R)-3-(2-methylthioethyl)-6-[(R)-1-(1-naphthyl)ethyl]piperazine-2,5-dione (0.68 g).

mp: 265—268°C.

IR (Nujol): 3200, 1675, 1660 $cm^{-1}$.

NMR ($CDCl_6$) δ: 0.38 (1H, m), 1.02 (1H, m), 1.49 (3H, d, J=8.1Hz), 1.69 (2H, t, J=8.1Hz), 1.73 (3H, s), 3.60 (1H, m), 4.19 (1H, s), 4.41 (1H, m), 7.51 (4H, m), 7.95 (3H, m), 8.30 (1H, m), 8.54 (1H, m).

## Example 6

The following compounds were prepared in a similar manner to that of Example 5 (6).

(1) (3R,6R)-3-(2-Ethoxycarbonylethyl)-6-(1-methylindol-3-ylmethyl)piperazine-2,5-dione

mp: 197—202°C.

IR (Nujol): 3180, 3050, 1730, 1670 $cm^{-1}$.

NMR (DMSO-$d_6$) δ: 1.14 (3H, t, J=6Hz), 1.25 (4H, m), 2.97 (1H, dd, J=14, 5Hz), 3.26 (1H, dd, J=14, 4Hz), 3.65 (1H, m), 3.96 (2H, q, J=6Hz), 4.15 (1H, m), 7.03 (1H, s), 7.05 (2H, m), 7.32 (1H, dd, J=7, 1Hz), 7.65 (1H, dd, J=7, 1Hz), 7.96 (1H, br s), 8.20 (1H, br s).

(2) (3R,6R)-3-[(R)-sec-Butyl]-6-(1-methylindol-3-ylmethyl)piperazine-2,5-dione

IR (Nujol): 3180, 3040, 1660 $cm^{-1}$.

NMR (DMSO-$d_6$) δ: 0.14 (3H, d, J=7Hz), 0.65 (3H, m), 1.00 (2H, m), 1.40 (1H, m), 3.05 (1H, dd, J=15, 5Hz), 3.25 (1H, dd, J=15, 4Hz), 3.63 (1H, m), 4.16 (1H, m), 7.04 (1H, s), 7.05 (2H, m), 7.35 (1H, dd, J=7, 1Hz), 7.65 (1H, dd, J=7, 1Hz), 7.75 (1H, s), 7.97 (1H, s).

(3) (3R,6R)-3-(1-Methylindol-3-ylmethyl)-6-(2-phenylthioethyl)piperazine-2,5-dione

mp: 176—182°C.

IR (Nujol): 3180, 1675, 1660 $cm^{-1}$.

NMR (DMSO-$d_6$) δ: 1.20 (2H, m), 1.9—2.2 (2H, m), 2.9—3.5 (6H, m), 4.15 (1H, m), 6.9—7.4 (9H, m), 7.60 (1H, m), 8.00 (1H, d, J=1Hz), 8.17 (1H, br s).

(4) (3R,6R)-3-Diphenylmethyl-6-(2-methylthioethyl)piperazine-2,5-dione

mp: 279—281°C.

IR (Nujol): 3200, 3050, 1665 $cm^{-1}$.

NMR (DMSO-$d_6$) δ: 1.05 (1H, m), 1.40 (1H, m), 1.93 (3H, s), 2.16 (2H, t, J=7.5Hz), 3.80 (1H, m), 4.60 (1H, d, J=4.5Hz), 4.80 (1H, m), 7.30 (10H, s), 7.96 (1H, d, J=1Hz), 8.16 (1H, d, J=1Hz).

(5) (3R,6R)-3-[(R)-α-Methoxybenzyl]-6-(2-methylthioethyl)piperazine-2,5-dione

mp: 253—258°C.

IR (Nujol): 3190, 3050, 1670, 1660 $cm^{-1}$.

(6) (3R,6R)-3,6-bis(1-Methylindol-3-ylmethyl)piperazine-2,5-dione
mp: 263—265°C.
NMR (DMSO-$d_6$) δ: 2.21 (2H, dd, J=14, 6Hz), 2.75 (2H, dd, J=14, 4.5Hz), 3.67 (6H, s), 3.90 (2H, m), 6.59 (2H, s), 6.9—7.5 (8H, m), 7.74 (2H, d, J=0.5Hz).

(7) (3R,6R)-3-(1-Methylindol-3-ylmethyl)-6-phenylthiomethylpiperazine-2,5-dione
mp: 216—219°C.
IR (Nujol): 3190, 3050, 1670 $cm^{-1}$.
NMR (DMSO-$d_6$) δ: 2.06 (1H, dd, J=13, 7Hz), 2.73 (1H, dd, J=13, 4Hz), 3.06 (1H, dd, J=14, 4Hz), 3.30 (1H, dd, J=14, 5Hz), 3.65 (3H, s), 4.83 (1H, m), 4.17 (1H, m), 6.9—7.4 (9H, m), 7.61 (1H, d, J=8Hz), 8.06 (1H, d, J=1Hz), 8.20 (1H, d, J=0.5Hz).

(8) (3R,6R)-3-(1-Methylindol-3-ylmethyl)-6-(3-methylthiopropyl)piperazine-2,5-dione
mp: 209—210°C.
IR (Nujol): 3180, 3040, 1675, 1660 $cm^{-1}$.
NMR (DMSO-$d_6$) δ: 0.55—1.2 (4H, m), 1.83 (3H, s), 1.98 (2H, t, J=7Hz), 2.97 (1H, dd, J=14, 5Hz), 3.26 (1H, dd, J=14, 4Hz), 3.55 (1H, m), 3.73 (3H, s), 4.14 (1H, m), 6.9—7.2 (2H, m), 7.03 (1H, s), 7.38 (1H, d, J=8Hz), 7.62 (1H, d, J=8Hz), 7.96 (1H, br s), 8.06 (1H, d, J=0.5Hz).

(9) (3R,6RS)-3-(2-Methylthioethyl)-6-(2-naphthylmethyl)piperazine-2,5-dione
mp: 179°C.
IR (Nujol): 3170, 3040, 1670 $cm^{-1}$.

(10) (3R,6R)-3-(4-Methoxybenzyl)-6-(2-methylthioethyl)piperazine-2,5-dione
mp: 266—267°C.
IR (Nujol): 3180, 1660 $cm^{-1}$.
NMR (DMSO-$d_6$) δ: 1.25 (2H, m), 1.88 (3H, s), 1.93 (2H, t, J=8Hz), 2.78 (1H, dd, J=14, 5Hz), 3.13 (1H, dd, J=14, 4Hz), 3.71 (3H, s), 3.75 (1H, m), 4.17 (1H, m), 6.85 (2H, d, J=9Hz), 7.11 (2H, d, J=9Hz), 8.08 (1H, s), 8.19 (1H, s).

(11) (3R,6R)-3-(2-Methylthioethyl)-6-(2-pyridylmethyl)piperazine-2,5-dione
mp: 163—165°C.
IR (Nujol): 3180, 3040, 1675 $cm^{-1}$.

(12) (3R,6R)-3-(2-Methylthioethyl)-6-(1-naphthylmethyl)piperazine-2,5-dione
mp: 236—240°C.
IR (Nujol): 3180, 3050, 1670 $cm^{-1}$.
NMR (DMSO-$d_6$) δ: 1.20 (2H, m), 1.86 (3H, s), 1.90 (2H, m), 3.49 (1H, m), 3.55 (1H, m), 3.71 (1H, m), 4.30 (1H, m), 7.50 (4H,m m), 7.90 (2H, m), 8.20 (3H, m).

(13) (3R,6R)-3-(1-Methylindol-3-ylmethyl)-6-methylthiomethylpiperazine-2,5-dione
mp: 244—247°C.

(14) (3R,6R)-3-Butyl-6-(1-methylindol-3-ylmethyl)piperazine-2,5-dione
mp: 237—239°C.
IR (Nujol): 3180, 3040, 1665 $cm^{-1}$.
NMR (DMSO-$d_6$) δ: 0.4—1.2 (9H, m), 3.00 (1H, dd, J=15, 5Hz), 3.25 (1H, dd, 15, 4Hz), 3.53 (1H, m), 3.70 (3H, s), 4.13 (1H, m), 7.01 (1H, s), 7.03 (2H, m), 7.33 (1H, dd, J=8, 1Hz), 7.62 (1H, dd, J=8, 1Hz), 7.89 (1H, br s), 8.01 (1H, br).

(15) (3R,6R)-3-(1-Methylindol-3-ylmethyl)-6-Octylpiperazine-2,5-dione
mp: 211—213°C.
IR (Nujol): 3170, 3040, 1660 $cm^{-1}$.
NMR (DMSO-$d_6$) δ: 0.5—1.4 (17H, m), 3.03 (1H, dd, J=15, 5Hz), 3.30 (1H, dd, J=15, 4Hz), 3.55 (1H, m), 3.73 (3H, s), 4.15 (1H, m), 7.03 (1H, s), 7.05 (2H, m), 7.35 (1H, dd, J=7, 1Hz), 7.63 (1H, dd, J=7, 1Hz), 7.92 (1H, br s), 8.03 (1H, br s).

(16) (3R,6R)-3-Dodecyl-6-(1-methylindol-3-ylmethyl)piperazine-2,5-dione
mp: 207—209°C.
IR (Nujol): 3160, 3030, 1660 $cm^{-1}$.
NMR (DMSO-$d_6$) δ: 0.4—1.4 (25H, m), 2.95 (1H, dd, J=14, 5Hz), 3.23 (1H, dd, J=14, 4Hz), 3.47 (1H, m), 3.68 (3H, s), 4.09 (1H, m), 7.00 (1H, s), 7.00 (2H, m), 7.30 (1H, dd, J=7, 1Hz), 7.58 (1H, dd, J=7, 1Hz), 7.87 (1H, br s), 8.00 (1H, br s).

(17) (3R,6R)-3-[(S)-1-(2-Naphthyl)ethyl]-6-(2-methylthioethyl)piperazine-2,5-dione
mp: 287—288°C.
IR (Nujol): 3190, 3140, 1660 $cm^{-1}$.
NMR (DMSO-$d_6$) δ: 0.60 (1H, m), 1.00 (1H, m), 1.46 (3H, d, J=8Hz), 1.48 (3H, s), 1.70 (2H, t, J=8Hz), 3.65 (2H, m), 4.10 (1H, m), 7.3—7.9 (7H, m), 8.01 (1H, m), 8.40 (1H, m).

(18) (3R,6R)-3-(2-Methylthioethyl)-6-[(RS)-1-phenylethyl]piperazine-2,5-dione
mp: 273—276°C.
IR (Nujol): 3180, 1660 $cm^{-1}$.
NMR (DMSO-$d_6$) δ: 0.60 (1H, m), 1.10 (1H, m), 1.33 (3H, d, J=7Hz), 1.83 (3H, s), 1.93 (2H, t, J=7.5Hz), 3.5 (2H, m), 3.96 (1H, m), 7.20 (5H, m), 8.00 (1H, br s), 8.30 (1H, br s).

(19) (3R,6R)-3-(3-Benzyloxycarbonylaminopropyl)-6-(1-methylindol-3-ylmethyl)piperazine-2,5-dione
mp: 170—172°C.
IR (Nujol): 3310, 3190, 1715, 1665 $cm^{-1}$.
NMR (DMSO-$d_6$) δ: 0.6—1.2 (4H, m), 2.60 (2H, m), 3.02 (1H, dd, J=15, 5Hz), 3.26 (1H, dd, J=15, 4Hz), 3.57 (1H, m), 3.70 (3H, s), 4.13 (1H, m), 5.00 (2H, s), 7.00 (4H, m), 7.30 (1H, m), 7.35 (5H, s), 7.60 (1H, d, J=7, 1Hz), 7.92 (1H, d, J=1Hz), 8.01 (1H, d, J=1Hz).

(20) (3R,6R)-3-(9-Anthrylmethyl)-6-(2-methylthioethyl)piperazine-2,5-dione
mp: 299—304°C.
IR (Nujol): 3180, 3050, 1660 $cm^{-1}$.
NMR (DMSO-$d_6$) δ: 1.56 (1H, m), 1.74 (1H, m), 2.02 (3H, s), 2.37 (2H, t, J=7.5Hz), 3.78 (1H, m), 4.02 (1H, dd, J=13.5, 7.5Hz), 4.12 (1H, dd, J=13.5, 5Hz), 7.52 (4H, m), 7.96 (1H, d, J=2.5Hz), 8.09 (2H, dd, J=8, 2Hz), 8.33 (2H, d, J=8Hz), 8.35 (1H, s), 8.55 (1H, s).

(21) (3R,6R)-3-(1-Methylindol-3-ylmethyl)-6-pentylpiperazine -2,5-dione
mp: 228°C.
IR (Nujol): 3190, 3050, 1680, 1670 $cm^{-1}$.
NMR (DMSO-$d_6$) δ: 0.5—1.2 :11H, m), 2.98 (1H, dd, J=15, 5Hz), 3.25 (1H, dd, J=15, 4Hz), 3.50 (1H, m), 3.70 (3H, s), 4.10 (1H, m), 6.8—7.2 (2H, m), 7.00 (1H, s), 7.31 (1H, dd, J=8, 1Hz), 7.60 (1H, dd, J=8, 1Hz), 7.89 (1H, br s), 7.99 (1H, br s).

(22) (3R,6R)-3-[(S)-1-(1-Naphthyl)ethyl]-6-(2-methylthioethyl)piperazine-2,5-dione
mp: 290—293°C.
IR (Nujol): 3180, 3040, 1660 $cm^{-1}$.

(23) (3R,6R)-3-(1-Methylindol-3-ylmethyl)-6-(2-phenylthioethyl)piperazine-2,5-dione
mp: 204—208°C.
IR (Nujol): 3180, 3050, 1675 $cm^{-1}$.

(24) (3R,6R)-3-(1-Methylindol-3-ylmethyl)-6-phenylthiomethylpiperazine-2,5-dione
mp: 213—214°C.
IR (Nujol): 3180, 3050, 1660 $cm^{-1}$.
NMR (DMSO-$d_6$) δ: 3.02 (1H, dd, J=14, 4.5Hz), 3.23 (3H, m), 3.50 (1H, m), 3.70 (3H, s), 4.20, (1H, m), 6.9—7.4 (3H, m), 7.05 (1H, s), 7.29 (5H, s), 7.62 (1H, dd, J=8, 1Hz), 7.95 (1H, s), 8.23 (1H, s).

(25) (3R,6S)-3-(1-Methylindol-3-ylmthyl)-6-(3-methylthiopropyl)piperazine-2,5-dione
mp: 205—206°C.
IR (Nujol): 3230, 1670, 1635 $cm^{-1}$.
NMR (DMSO-$d_6$) δ: 1.59 (4H, m), 2.00 (3H, s), 2.38 (2H, t, J=7Hz), 2.95—3.5 (3H, m), 3.75 3H, s), 4.13 (1H, m), 6.9—7.3 (2H, m), 7.10 (1H, s), 7.42 (1H, d, J=8Hz), 7.65 (1H, d, J=8Hz), 7.98 (1H, s), 8.05 (1H, d, J=1Hz).

(26) (3R,6S)-3-(1-Methylindol-3-ylmethyl)-6-octylpiperazine-2,5-dione
mp: 201—202°C.
IR (Nujol): 3170, 3040, 1665 $cm^{-1}$.
NMR (DMSO-$d_6$) δ: 0.86 (3H, m), 1.20 (12H, m), 1.53 (2H, m), 3.15 (3H, m), 3.72 (3H, s), 4.10 (1H, m), 7.05 (2H, m), 7.06 (1H, s), 7.36 (1H, dd, J=7, 1Hz), 7.60 (1H, dd, J=7, 1Hz), 7.88 (1H, br s), 7.98 (1H, br s).

(27) (3R,6R)-3-Dodecyl-6-(1-methylindol-3-ylmethyl)piperazine-2,5-dione
mp: 187—188°C.
IR (Nujol): 3160, 1670, 1660 $cm^{-1}$.
NMR (DMSO-$d_6$) δ: 0.8—1.6 (25H, m), 3.1 (3H, m), 3.70 (3H, s), 4.05 (1H, m), 6.9—8.0 (7H, m).

(28) (3R,6R)-3-(3-Benzyloxycarbonylaminopropyl)-6-(1-methylindol-3-ylmethyl)piperazine-2,5-dione
mp: 128—130°C.
IR (Nujol): 3330, 3190, 1670 $cm^{-1}$.
NMR (DMSO-$d_6$) δ: 1.43 (4H m), 2.8—3.3 (5H, m), 3.70 (3H, s), 4.05 (1H, m), 4.97 (2H, s), 7.03 (4H, m), 7.30 (5H, s), 7.30 (1H, m), 7.58 (1H, dd, J=8, 1Hz), 7.87 (1H, s), 8.00 (1H, d, J=1Hz).

(29) (3R,6RS)-3-(1-Methylindol-3-ylmethyl)-6-pentylpiperazine-2,5-dione
mp: 204—212°C.
IR (Nujol): 3200, 1680 $cm^{-1}$.
NMR (DMSO-$d_6$) δ: 0.80 (3H, m), 1.15 (6H, m), 1.46 (1H, m), 2.9—3.3 (3H, m), 3.70 (3H, s), 4.07 (1H, m), 6.85—7.2 (2H, m), 7.02 (1H, s), 7.33 (1H, dd, J=7, 2Hz), 7.57 (1H, dd, J=7, 2Hz), 7.86 (1H, s), 7.92 (1H, br s).

(30) (3R,6R)-3-(1-Methyl-2-phenylindol-3-ylmethyl)-6-(2-methylthioethyl)piperazine-2,5-dione
mp: 295—298°C.
IR (Nujol): 3200, 1670, 1660°C.
NMR (DMSO-$d_6$) δ: 1.0—1.6 (2H, m), 1.90 (3H, s), 2.03—2.90 (2H, m), 2.8—3.1 (2H, m), 3.50 (3H, s), 3.5—4.0 (2H, m), 7.0—8.1 (11H, m).

(31) (3R,6R)-3-(1-Methylindol-3-ylmethyl)-6-propylpiperazine-2,5-dione
mp: 241—243°C.
IR (Nujol): 3200, 1665, 1660 $cm^{-1}$.
NMR (DMSO-$d_6$) δ: 0.3—1.2 (7H, m), 3.0—3.3 (2H, m), 3.4—3.6 (1H, m), 3.70 (3H, s), 4.0—4.2 (1H, m), 6.9—8.1 (7H, m).

(32) (3R,6R)-3-Butyl-6-[(R)-1-(1-naphthyl)ethyl]piperazine-2,5-dione
mp: 279—281°C.
IR (Nujol): 3200, 1670, 1660 $cm^{-1}$.
NMR (DMSO-$d_6$) δ: −0.21—0.0 (1H, m), 0.34—0.82 (8H, m), 1.47 (3H, d, J=7.9Hz), 3.32 (1H, m), 4.11 (1H, s), 4.34 (1H, m), 7.39 (4H, m), 7.76 (3H, m), 8.16 (1H, m), 8.42 (1H, m).

(33) (3R,6R)-3-(1-Methylindol-3-ylmethyl)-6-benzylpiperazine-2,5-dione
mp: 283—284°C.
IR (Nujol): 3110, 1660 $cm^{-1}$.
NMR (DMSO-$d_6$) δ: 2.00 (1H, dd, J=13.5, 6Hz), 2.37 (1H, dd, J=14.5, 6Hz), 2.46 (1H, dd, J=13.5, 6Hz), 2.77 (1H, dd, J=14.5, 4Hz), 3.45 (1H, m), 3.90 (1H, m), 6.78 (2H, m), 6.97 (1H, s), 7.0—7.25 (5H, m), 7.38 (1H, d, J=8Hz), 7.48 (1H, d, J=8Hz), 7.80 (1H, br s), 7.93 (1H, br s).

(34) (3R,6R)-3-(1-Methylindol-3-ylmethyl)-6-phenethylpiperazine-2,5-dione
mp: 240—242°C.
IR (Nujol): 3170, 1660 $cm^{-1}$.
NMR (DMSO-$d_6$) δ: 0.97 (1H, m), 1.25 (1H, m), 1.79 (2H, m), 3.00 (1H, dd, J=15, 4.5Hz), 3.30 (1H, dd, J=15, 4Hz), 3.53 (3H, s), 3.62 (1H, m), 4.27 (1H, br s), 6.77 (2H, d, J=7.5Hz), 7.0—7.3 (7H, m), 7.65 (1H, d, J=7.5Hz), 8.03 (1H, s), 8.17 (1H, s).

(35) (3R,6S)-3-(1-Methylindol-3-ylmethyl)-6-phenethylpiperazine-2,5-dione
mp: 218—220°C.
IR (Nujol): 3170, 1660 $cm^{-1}$.
NMR (DMSO-$d_6$) δ: 1.81 (2H, m), 2.50 (2H, m), 3.0—3.3 (2H, m), 3.71 (3H, s), 4.10 (1H, m), 7.0—7.3 (8H, m), 7.36 (1H, d, J=8Hz), 7.60 (1H, d, J=8Hz), 8.08 (1H, s), 8.11 (1H, s).

(36) (3R,6RS)-3-(2-Methylthioethyl)-6-phenethylpiperazine-2,5-dione
mp: 231—233°C.
NMR (DMSO-$d_6$) δ: 1.98 (4H, m), 2.05 (3H, s), 2.55 (4H, m), 3.90 (1H, m), 4.00 (1H, m), 7.25 (5H, m), 8.28 (1H, s), 8.35 (1H, s).

(37) (3R,6R)-3-Hydroxymethyl-6-(1-methylindol-3-ylmethyl)piperazine-2,5-dione
mp: 233—235°C.
IR (Nujol): 3150, 1670 $cm^{-1}$.
NMR (DMSO-$d_6$) δ: 3.2 (4H, m), 3.7 (1H, m), 3.73 (3H, s), 4.02 (1H, m), 4.92 (1H, t, J=5Hz), 7.1 (3H, m), 7.40 (1H, dd, J=7, 2Hz), 7.57 (1H, dd, J=7, 2Hz), 7.85 (2H, m), 7.38 (1H, d, J=8Hz), 7.58 (1H, d, J=8Hz), 8.16 (1H, m), 8.22 (1H, m).

(38) (3R,6R)-3-(2-Benzyloxycarbonylethyl)-6-(1-methylindol-3-ylmethyl)piperazine-2,5-dione
mp: 207—208°C.
IR (Nujol): 3160, 1720, 1644 $cm^{-1}$.
NMR (DMSO-$d_6$) δ: 1.15 (2H, m), 1.25 (2H, m), 2.97 (1H, dd, J=14, 4.5Hz), 3.28 (1H, dd, J=14, 3.5Hz), 3.60 (3H, s), 3.68 (1H, m), 4.15 (1H, m), 4.99 (2H, s), 6.92—7.48 (9H, m), 7.61 (1H, d, J=8Hz), 7.97 (1H, s), 8.22 (1H, s).

(39) (3R,6R)-3-Benzyloxymethyl-6-(1-methylindol-3-ylmethyl)piperazine-2,5-dione
mp: 210—211°C.
IR (Nujol): 3170, 1660 $cm^{-1}$.
NMR (DMSO-$d_6$) δ: 2.71 (1H, dd, J=10, 5Hz), 3.05 (1H, dd, J=14.5, 4.5Hz), 3.18 (1H, dd, J=14.5, 5Hz), 3.26 (1H, dd, J=10, 3Hz), 3.68 (3H, s), 3.81 (1H, m), 4.06 (1H, m), 4.15 (1H, d, J=13Hz), 4.23 (1H, d, J=13Hz), 6.94—7.50 (10H, m), 8.05 (2H, s).

(40) (3R,6R)-3-(1-Methylindol-3-ylmethyl)-6-(2-pyridylmethyl)piperazine-2,5-dione
mp: 188—192°C.

(41) (3R,6S)-3-(1-Methylindol-3-ylmethyl)-6-(2-pyridylmethyl)piperazine-2,5-dione
mp: 197—201°C.

(42) (3R,6R)-3-[(S)-1-(1-Methylindol-3-yl)ethyl]-6-(2-methylthioethyl)piperazine-2,5-dione
mp: 264—267°C.

(43) (3R,6R)-3-[(R)-1-(1-Methylindol-3-yl)ethyl]-6-(2-methylthioethyl)piperazine-2,5-dione
mp: 279—280°C.

(44) (3R,6R)-3-Butyl-6-[(R)-1-(1-methylindol-3-yl)ethyl]piperazine-2,5-dione
mp: 285—288°C.

(45) (3R,6S)-3-(1-Methylindol-3-ylmethyl)-6-methylthiomethylpiperazine-2,5-dione
mp: >260°C.

Example 7

(1) To a mixture of 2-methylindole (4.26 g) and methyl (2R,3R)-N-benzyloxycarbonyl-3-methyl-2-aziridinecarboxylate (2.70 g) in dry methylene chloride (50 ml) was added boron trifluoride etherate (1.33 ml) under ice-bath cooling. The mixture was stirred for five minutes at ambient temperature and the solvent was evaporated off. The residue was chromatographed on silica gel (chloroform and a 1:1 mixture of hexane and ether as eluent) to give (αR,βR)-N-benzyloxycarbonyl-1β-dimethyltryptophane methyl ester (1.62 g) as an oil.
IR (Neat): 1715, 1670 $cm^{-1}$.

(2) A solution of (αR,βR)-N-benzyloxycarbonyl-1,β-dimethyltryptophane methyl ester (1.62 g) in methanol (50 ml) containing 10% paradium charcoal (300 mg) was hydrogenated at the ambient temperature and atmospheric pressure. After removal of the catalyst and evaporation of the solvent, the residual oil (1.13 g) was dissolved in methanol (5 ml) and to the solution, a solution of p-toluenesulfonic acid mono hydrate (0.81 g) in a mixture of water (2 ml) and methanol (2 ml) was added. The solution was evaporated to dryness to give p-toluenesulfonic acid salt of (αR,βR)-1,β-dimethyltryptophane methyl ester (1.77 g).
IR (Neat): 3200, 1715 $cm^{-1}$.

(3) The following compound was prepared in a similar manner to that of Example 5 (1).
N-[N-tert-Butoxycarbonyl-D-leucyl]-(αR,βR)-1,β-dimethyltryptophane methyl ester
IR (Nujol): 1715, 1670 $cm^{-1}$.

(4) The following compound was prepared in a similar manner to that of Example 5 (2).
(3R,6R)-3-Isobutyl-6-[(R)-1-(1-methylindol-3-yl)ethyl]piperazine-2,4-dione
IR (Nujol): 3150, 1680 $cm^{-1}$.

Example 8

A solution of (3R,6R)-3-(3-benzyloxycarbonylaminopropyl)-6-(1-methylindol-3-ylmethyl)piperazine-2,5-dione (1.2 g) in ethanol (240 ml) containing 10% paradium on charcoal (0.6 g) was hydrogenated at ambient temperature and atmospheric pressure. After catalyst was filtered off and solvent was evaporated, crude solid was taken up with ethanol, and dried in vacuo to give (3R,6R)-3-(3-aminopropyl)-6-(1-methylindol-3-ylmethyl)piperazine-2,5-dione.
mp: 148°C.

Example 9

The following compound was prepared in a similar manner to that of Example 8.

(3S,6R)-3-(3-Aminopropyl)-6-(1-methylindol-3-ylmethyl)piperazine-2,5-dione

mp: 175—177°C.

Example 10

To a solution of (3R,6R)-3-(3-aminopropyl)-6-(1-methylindol-3-ylmethyl)piperazine-2,5-dione (100 mg) in dry N,N-dimethylformamide (3 ml) was added benzoyl chloride (60 mg). The mixture was stirred at ambient temperature for 2 hours, diluted with water (15 ml) and allowed to stand overnight. The precipitated mass was collected, washed with water, and dried in vacuo to give (3R,6R)-3-(3-benzamidopropyl)-6-(1-methylindol-3-ylmethyl)piperazine-2,5-dione (45 mg).

mp: 238—239°C.

IR (Nujol): 3320, 1675, 1630 $cm^{-1}$.

NMR (DMSO-$d_6$) δ: 0.85 (3H, m), 1.08 (1H, m), 2.87 (2H, m), 3.00 (1H, dd, J=14, 4.5Hz), 3.23 (1H, dd, J=14, 4Hz), 3.59 (1H, m), 3.70 (3H, s), 4.11 (1H, m), 7.05 (3H, m), 7.29 (1H, d, J=8Hz), 7.45 (3H, m), 7.58 (1H, d, J=8Hz), 7.80 (2H, m), 7.99 (1H, s), 8.09 (1H, s), 8.24 (1H, t, J=5.5Hz).

Example 11

A mixture of (3R,6R)-3-hydroxymethyl-6-(1-methylindol-3-ylmethyl)-piprazine-2,5-dione (0.10 g), dry pyridine (1.0 ml) and acetic anhydride (0.08 g) was heated at 100°C for 1 hour. After the mixture was cooled to ambient temperature, the precipitated mass was collected, washed with pyridine and then ethanol, and dried to give (3R,6R)-3-acetoxymethyl-6-(1-methylindol-3-ylmethyl)piperazine-2,5-dione (61.8 mg).

mp: 250—252°C.

IR (Nujol): 3180, 1735, 1670, 1660 $cm^{-1}$.

NMR (DMSO-$d_6$) δ: 1.85 (3H, s), 3.10 (1H, dd, J=15, 4.5Hz), 3.18 (1H, dd, J=15, 4.5Hz), 3.23 (1H, dd, J=10.5, 5.5Hz), 3.73 (3H, s), 3.81 (1H, dd, J=10.5, 3.5Hz), 3.98 (1H, m), 4.11 (1H, m), 7.00 (1H, dd, J=8, 7Hz), 7.09 (1H, s), 7.12 (1H, t, J=8, 7Hz).

Example 12

A mixture of (3R,6R)-3-(3-aminopropyl)-6-(1-methylindol-3-ylmethyl)piperazine-2,5-dione (0.1 g) and acetic anhydride (0.08 g) in pyridine (1.0 ml) was heated at 100°C for 1 hour. After the mxiture was cooled to ambient temperature, the precipitated solid was collected by filtration and washed with pyridine (2 ml) and ethanol (5 ml) to give (3R,6R)-3-(3-acetamidopropyl)-6-(1-methylindol-3-ylmethyl)piperazine-2,5-dione (0.1 g).

mp: 138°C.

IR (Nujol): 3190, 1660 $cm^{-1}$.

NMR (DMSO-$d_6$) δ: 0.70 (3H, m), 1.00 (1H, m), 1.75 (3H, s), 2.40 (2H, m), 3.00 (1H, dd, J=13Hz, 4.5Hz), 3.22 (1H, dd, J=13Hz, 4Hz), 3.52 (1H, m), 3.74 (3H, s), 4.11 (1H, m), 7.05 (3H, m), 7.33 (1H, d, J=8Hz), 7.58 (1H, d, J=8Hz), 7.96 (1H, s), 8.05 (1H, s).

Example 13

The following compound was prepared in a similar manner to that of Example 12.

(3R,6R)-3-(3-Acetamidopropyl)-6-(1-methylindol-3-ylmethyl)piperazine-2,5-dione

mp: 124—126°C.

IR (Nujol): 3280, 3180, 1670 $cm^{-1}$.

NMR (DMSO-$d_6$) δ: 1.30 (2H, m), 1.48 (2H, m), 1.75 (3H, s), 2.93 (2H, m), 3.10 (1H, m), 3.15 (1H, m), 3.22 (1H, m), 3.72 (3H, s), 4.05 (1H, m), 7.05 (3H, m), 7.35 (1H, d, J=8Hz), 7.60 (1H, d, J=8Hz), 7.75 (1H, m).

Example 14

To a solution of (3R,6R)-3-(2-methylthioethyl)-6-[(R)-1-(1-naphthyl)ethyl]piperazine-2,5-dione (0.40 g) in dry dimethylformamide (40 ml) was added sodium hdyride (116 mg). After the mixture was stirred at ambient temperature for 10 minutes, methyl iodide (0.19 ml) was added to the mixture. The reaction mixture was stirred for 5 minutes and the solvent was evaporated off under reduced pressure. The residue was extracted with chloroform (40 ml). The organic extract was washed with water, dried over magnesium sulfate, and evaporated in vacuo. The residue was chromatographed on silica gel (8 g) using a mixture of chloroform and methanol as eluent to give (3R,6R)-3-1,4-dimethyl-3-(2-methylthioethyl)-6-[(R)-1-(1-naphthyl)ethyl]piperazine-2,5-dione (0.16 g).

mp: 88—90°C (recrystallized from isopropyl ether).

IR (Chloroform): 1655 $cm^{-1}$.

NMR ($CDCl_3$) δ: 0.7—1.3 (2H, m), 1.63 (3H, d, J=6Hz), 2.03 (1H, s), 2.50 (2H, t, J=6Hz), 2.77 (3H, s), 2.80 (3H, s), 3.77 (1H, m), 4.30 (2H, m), 7.3—8.2 (7H, m).

Example 15

The following compounds were prepared in a similar manner to that of Example 13.

(1) (3R,6R)-3-Butyl-1,4-dimethyl-6-[(R)-1-(1-naphthyl)ethyl]piperazine-2,5-dione
mp: 71—73°C.
IR (Nujol): 1650 $cm^{-1}$.
NMR ($CDCl_3$) δ: 0.88 (3H, m), 1.25 (6H, m), 1.63 (3H, d, J=7Hz), 2.60 (3H, s), 2.83 (3H, s), 3.56 (1H, m), 4.23 (1H, m), 4.28 (1H, m), 7.4—8.2 (7H, m).

(2) (3R,6R)-3-Butyl-1,4-dimethyl-6-[(R)-1-(1-methylindol-3-yl)ethyl)piperazine-2,5-dione
IR (Neat): 1650 $cm^{-1}$.

(3) (3R,6R)-1,4-Dimethyl-3-[(R)-1-(1-methylindol-3-yl)ethyl]-6-(2-mthylthioethyl)piperazine-2,5-dione
IR ($CHCl_3$): 1650 $cm^{-1}$.

(4) (3R,6R)-1,4-Dimethyl-3-[(S)-1-(1-methylindol-3-yl)ethyl]-6-(2-mthylthioethyl)piperazine-2,5-dione
IR ($CHCl_3$): 1650 $cm^{-1}$.

Example 16

A solution of (3R,6R)-3-(2-benzyloxycarbonylethyl)-6-(1-methylindol-3-ylmethyl)piperazine-2,5-dione (1.50 g) in ethanol (300 ml) containing 10% paradium on charcoal (0.75 g) was hydrogenated at ambient temperature and atmospheric pressure. After catalyst was filtered off and solvent was evaporated, crude solid was recrystallized from ethanol and dried in vacuo to give (3R,6R)-3-(2-carboxyethyl)-6-(1-methylindol-3-ylmethyl)piperazine-2,5-dione (0.58 g).
mp: 228°C.
IR (Nujol): 1720, 1660 $cm^{-1}$.
NMR (DMSO-$d_6$) δ: 1.14 (3H, m), 1.38 (1H, m), 2.98 (1H, dd, J=14, 4.5Hz), 3.23 (1H, dd, J=14, 4Hz), 3.63 (1H, m), 3.70 (3H, s), 4.12 (1H, m), 7.00 (3H, m), 7.28 (1H, d, J=8Hz), 7.59 (1H, d, J=8Hz), 7.97 (1H, s), 8.14 (1H, s).

Example 17

| | |
|---|---|
| (3R,6R)-3-(2-Methylthioethyl)-6-[(R)-1-(1-naphthyl)ethyl]piperazine-2,5-dione | 500 (g) |
| Sucrose | 9000 |
| Hydroxypropylcellulose | 250 |
| Starch | 250 |

The above ingredients are blended and granulated or grained in a conventional manner into granules or small granules.

## Claims

1. A piperazine compound of the formula:

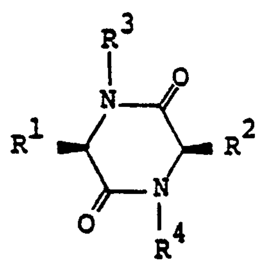

(Ia)

wherein

$R^1$ is phenyl); mono(or di)phenyl($C_1$—$C_6$)alkyl; naphthyl($C_1$—$C_6$)alkyl; anthryl($C_1$—$C_6$)alkyl; phenyl($C_1$—$C_6$)alkyl having $C_1$—$C_6$ alkoxy substituent(s); indolyl($C_1$—$C_6$)alkyl; benzothienyl($C_1$—$C_6$)alkyl; pyridyl($C_1$—$C_6$)alkyl; N-$C_1$—$C_6$ alkylindolyl($C_1$—$C_6$)alkyl; or N-$C_1$—$C_6$ alkylindolyl($C_1$—$C_6$)alkyl having phenyl substituent(s) on the indole ring;

$R^2$ is $C_1$—$C_6$ alkyl; $C_7$—$C_{22}$ alkyl; $C_1$—$C_6$ alkylthio($C_1$—$C_6$)alkyl; $C_2$—$C_6$ alkenylthio($C_1$—$C_6$)alkyl; hydroxy($C_1$—$C_6$)alkyl; $C_1$—$C_6$ alkanoyloxy($C_1$—$C_6$)alkyl; phenyl($C_1$—$C_6$)alkoxy($C_1$—$C_6$)alkyl; amino($C_1$—$C_6$)-alkyl; $C_1$—$C_6$ alkanoylamino($C_1$—$C_6$)alkyl; benzamido($C_1$—$C_6$)alkyl; phenyl($C_1$—$C_6$)alkoxycarbonyl-

amino($C_1$—$C_6$)alkyl; carboxy($C_1$—$C_6$)alkyl; $C_1$—$C_6$ alkoxycarbonyl($C_1$—$C_6$)alkyl; phenyl($C_1$—$C_6$)alkoxycarbonyl($C_1$—$C_6$)alkyl; phenylthio($C_1$—$C_6$)alkyl; phenyl($C_1$—$C_6$)alkylthio($C_1$—$C_6$)alkyl; or N—$C_1$—$C_6$ alkylindolyl($C_1$—$C_6$)alkyl, and

$R^3$ and $R^4$ are each hydrogen or $C_1$—$C_6$ alkyl, or pharmaceutically acceptable salts thereof.

2. A compound of claim 1, in which

$R^1$ is phenyl; benzyl; phenethyl; diphenylmethyl; 1-phenylethyl; 1-naphthylmethyl; 2-naphthylmethyl; 1-(1-naphthyl)ethyl; 1-(2-naphthyl)ethyl; 9-anthrylmethyl; α-methoxybenzyl; 4-methoxybenzyl) 3-indolylmethyl; benzo[b]thien-3-yl; 2-pyridylmethyl; 1-methylindol-3-ylmethyl; 1-(1-methylindol-3-yl)ethyl; or 1-methyl-2-phenylindol-3-ylmethyl;

$R^2$ is butyl; isobutyl; sec-butyl; pentyl; octyl; dodecyl; methylthiomethyl; 2-methylthioethyl; 3-methylthiopropyl; butylthiomethyl; allylthiomethyl; hydroxymethyl; acetoxymethyl; benzyloxymethyl; 3-aminopropyl; 3-acetamidopropyl; 3-benzamidopropyl; 3-benzyloxycarbonylaminopropyl; 2-carboxyethyl; 2-ethoxycarbonylethyl; 2-benzyloxycarbonylethyl; phenylthiomethyl; 2-phenylthioethyl; benzylthiomethyl; or 1-methylindol-3-ylmethyl; and

$R^3$ and $R^4$ are each hydrogen or methyl.

3. A compound of claim 1, in which

$R^3$ and $R^4$ are hydrogen or $C_1$—$C_6$ alkyl;

$R^1$ is mono(or di)phenyl($C_1$—$C_6$)alkyl; naphthyl($C_1$—$C_6$)alkyl; anthryl($C_1$—$C_6$)alkyl; N—$C_1$—$C_6$ alkylindolyl($C_1$—$C_6$)alkyl; or N—$C_1$—$C_6$ alkylindolyl($C_1$—$C_6$)alkyl having phenyl on the indole ring; and

$R^2$ is $C_1$—$C_6$ alkyl; $C_1$—$C_6$ alkylthio($C_1$—$C_6$)alkyl; or N—$C_1$—$C_6$ alkylindolyl($C_1$—$C_6$)alkyl.

4. A compound of claim 3, which is (3R,6R)-3-(2-methylthioethyl)-6-[(R)-1-(1-naphthyl)ethyl]piperazine-2,5-dione or a pharmaceutically acceptable salt thereof.

5. A process for preparing a piperazine compound of the formula:

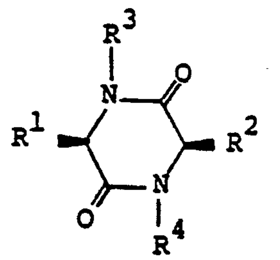

(Ia)

wherein

$R^1$ is phenyl); mono(or di)phenyl($C_1$—$C_6$)alkyl; naphthyl($C_1$—$C_6$)alkyl; anthryl($C_1$—$C_6$)alkyl; phenyl($C_1$—$C_6$)alkyl having $C_1$—$C_6$ alkoxy substituent(s); indolyl($C_1$—$C_6$)alkyl; benzothienyl($C_1$—$C_6$)alkyl; pyridyl($C_1$—$C_6$)alkyl; N-$C_1$—$C_6$ alkylindolyl($C_1$—$C_6$)alkyl; or N-$C_1$—$C_6$ alkylindolyl($C_1$—$C_6$)alkyl having phenyl substituent(s) on the indole ring;

$R^2$ is $C_1$—$C_6$ alkyl; $C_7$—$C_{22}$ alkyl; $C_1$—$C_6$ alkylthio($C_1$—$C_6$)alkyl; $C_2$—$C_6$ alkenylthio($C_1$—$C_6$)alkyl; hydroxy($C_1$—$C_6$)alkyl; $C_1$—$C_6$ alkanoyloxy($C_1$—$C_6$)alkyl; phenyl($C_1$—$C_6$)alkoxy($C_1$—$C_6$)alkyl; amino($C_1$—$C_6$)alkyl; $C_1$—$C_6$ alkanoylamino($C_1$—$C_6$)alkyl; benzamido($C_1$—$C_6$)alkyl; phenyl($C_1$—$C_6$)alkoxycarbonylamino($C_1$—$C_6$)alkyl; carboxy($C_1$—$C_6$)alkyl; $C_1$—$C_6$ alkoxycarbonyl($C_1$—$C_6$)alkyl; phenyl($C_1$—$C_6$)alkoxycarbonyl($C_1$—$C_6$)alkyl; phenylthio($C_1$—$C_6$)alkyl; phenyl($C_1$—$C_6$)alkylthio($C_1$—$C_6$)alkyl; or N—$C_1$—$C_6$ alkylindolyl($C_1$—$C_6$)alkyl, and

$R^3$ and $R^4$ are each hydrogen or $C_1$—$C_6$ alkyl, or a salt thereof, which comprises,

(a) subjecting a compound of the formula:

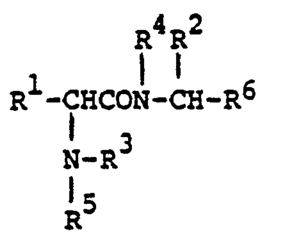

(II)

wherein

$R^1$, $R^2$, $R^3$ and $R^4$ are each as defined above,

$R^5$ is hydrogen or an amino-protective group, and

$R^6$ is carboxy or protected carboxy,

or its reactive derivative at the carboxy or amino group, or a salt rhereof, to a ring closure reaction, to give a compound of the formula:

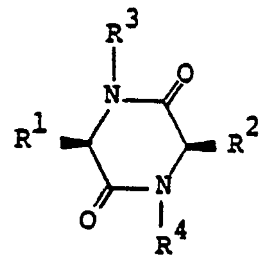

(Ia)

wherein

$R^1$, $R^2$, $R^3$ and $R^4$ are each as defined above, or a salt thereof;

(b) reacting a compound of the formula:

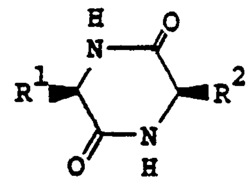

(Ib')

wherein

$R^1$ and $R^2$ are each as defined above,

or a salt thereof, with a $C_1$—$C_6$ alkylating agent, to give a compound of the formula:

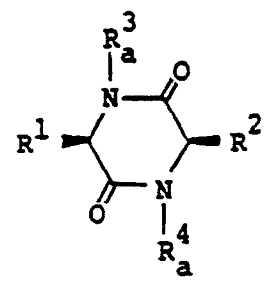

(Ic')

wherein

$R^1$ and $R^2$ are each as defined above, and

$R^3_a$ and $R^4_a$ are each $C_1$—$C_6$ alkyl,

or a salt thereof;

(c) reacting a compound of the formula:

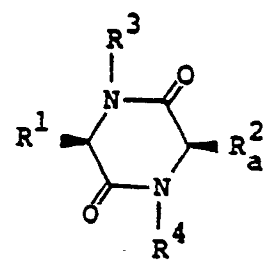

(Id')

wherein

$R^1$, $R^3$ and $R^4$ are each as defined above, and

$R^2_a$ is amino($C_1$—$C_6$)alkyl,

or its reactive derivative at the amino group, or a salt thereof, with an acylating agent, to give a compound of the formula:

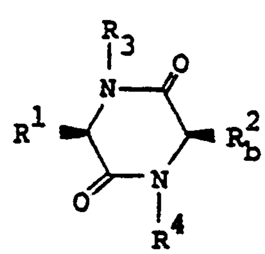

(Ie')

wherein
$R^1$, $R^3$ and $R^4$ are each as defined above, and
$R^2_b$ is $C_1$—$C_6$ alkanoylamino($C_1$—$C_6$)alkyl,
or a salt thereof;
(d) reacting a compound of the formula:

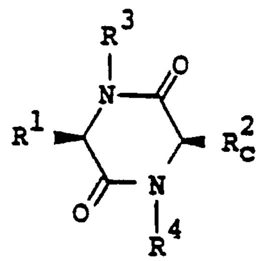

(If')

wherein
$R^1$, $R^3$ and $R^4$ are each as defined above, and
$R^2_c$ is hydroxy($C_1$—$C_6$)alkyl,
or a salt thereof, with an acylating agent, to give a compound of the formula:

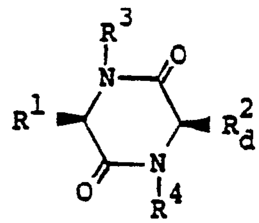

(Ig')

wherein
$R^1$, $R^3$ and $R^4$ are each as defined above, and
$R^2_d$ is $C_1$—$C_6$ alkanoyloxy($C_1$—$C_6$)alkyl,
or a salt thereof;
(e) subjecting a compound of the formula:

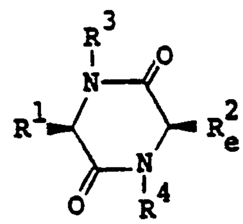

(Ih')

wherein
$R^1$, $R^3$ and $R^4$ are each as defined above, and
$R^2_e$ is a protected amino($C_1$—$C_6$)alkyl,
or a salt thereof, to a removal reaction of the amino-protective group, to give a compound of the formula:

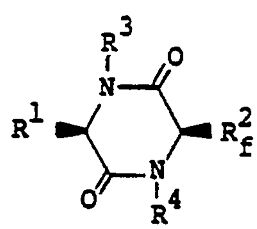

(Ii')

wherein
$R^1$, $R^3$ and $R^4$ are each as defined above, and
$R^2_f$ is amino($C_1$—$C_6$)alkyl
or a salt thereof; or

(f) subjecting a compound of the formula:

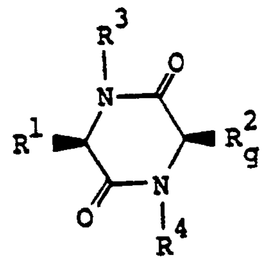

(Ij')

wherein

$R^1$, $R^3$ and $R^4$ are each as defined above, and

$R^2_g$ is a protected carboxy($C_1$—$C_6$)alkyl,

or a salt thereof, to a removal reaction of the carboxy-protective group to give a compound of the formula:

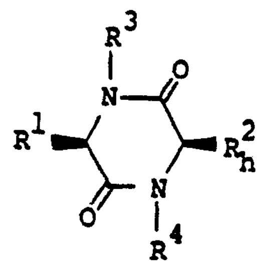

(Ik')

wherein

$R^1$, $R^3$ and $R^4$ are each as defined above, and

$R^2_h$ is carboxy($C_1$—$C_6$)alkyl,

or a salt thereof.

6. A pharmaceutical composition comprising, as an effective ingredient, a piperazine compound of the formula:

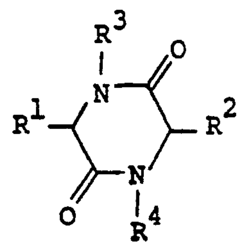

(I)

wherein

$R^1$ is phenyl); mono(or di)phenyl($C_1$—$C_6$)alkyl; naphthyl($C_1$—$C_6$)alkyl; anthryl($C_1$—$C_6$)alkyl; phenyl($C_1$—$C_6$)alkyl having $C_1$—$C_6$ alkoxy substituent(s); indolyl($C_1$—$C_6$)alkyl; benzothienyl($C_1$—$C_6$)alkyl; pyridyl($C_1$—$C_6$)alkyl; N-$C_1$—$C_6$ alkylidonyl($C_1$—$C_6$)alkyl; or N-$C_1$—$C_6$ alkylindolyl($C_1$—$C_6$)alkyl having phenyl substituent(s) on the indole ring;

$R^2$ is $C_1$—$C_6$ alkyl; $C_7$—$C_{22}$ alkyl; $C_1$—$C_6$ alkylthio($C_1$—$C_6$)alkyl; $C_2$—$C_6$ alkenylthio($C_1$—$C_6$)alkyl; hydroxy($C_1$—$C_6$)alkyl; $C_1$—$C_6$ alkanoyloxy($C_1$—$C_6$)alkyl; phenyl($C_1$—$C_6$)alkoxy($C_1$—$C_6$)alkyl; amino($C_1$—$C_6$)-alkyl; $C_1$—$C_6$ alkanoylamino($C_1$—$C_6$)alkyl; benzamido($C_1$—$C_6$)alkyl; phenyl($C_1$—$C_6$)alkoxycarbonyl-amino($C_1$—$C_6$)alkyl; carboxy($C_1$—$C_6$)alkyl; $C_1$—$C_6$ alkoxycarbonyl($C_1$—$C_6$)alkyl; phenyl($C_1$—$C_6$)alkoxy-carbonyl($C_1$—$C_6$)alkyl; phenylthio($C_1$—$C_6$)alkyl; phenyl($C_1$—$C_6$)alkylthio($C_1$—$C_6$)alkyl; or N—$C_1$—$C_6$ alkyl-indolyl($C_1$—$C_6$)alkyl, and

$R^3$ and $R^4$ are each hydrogen or $C_1$—$C_6$ alkyl, or pharmaceutically acceptable salts thereof, and a pharmaceutically acceptable carrier.

7. A piperazine compound of the formula:

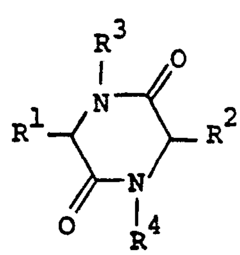

(I)

wherein $R^1$, $R^2$, $R^3$ and $R^4$ are each as defined in claim 6, or a pharmaceutically acceptable salt thereof, for use as a PAF antagonist.

8. A process for preparing a pharmaceutical composition which comprises mixing together a piperazine compound of the formula:

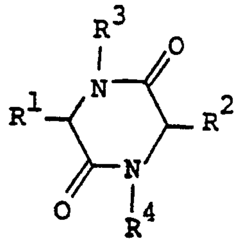

(I)

wherein $R^1$, $R^2$, $R^3$ and $R^4$ are each as defined in claim 6, or a pharmaceutically acceptable salt thereof, and a pharmaceutically acceptable carrier.

**Patentansprüche**

1. Eine Piperazinverbindung der Formel:

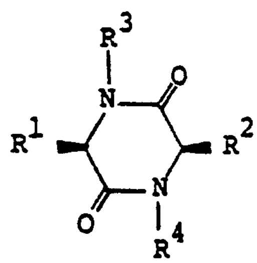

(Ia)

worin

$R^1$ Phenyl; Mono(oder Di)phenyl($C_1$—$C_6$)alkyl; Naphthyl($C_1$—$C_6$)alkyl; Anthryl($C_1$—$C_6$)alkyl; Phenyl($C_1$—$C_6$)alkyl mit $C_1$—$C_6$ Alkoxy-Substituent(en); Indolyl($C_1$—$C_6$)alkyl; Benzothienyl($C_1$—$C_6$)alkyl; Pyridyl($C_1$—$C_6$)alkyl; N—$C_1$—$C_6$-Alkylindolyl($C_1$—$C_6$)alkyl; oder N—$C_1$—$C_6$-Alkylindolyl($C_1$—$C_6$)alkyl mit Phenyl-Substituent(en) am Indolring ist;

$R^2$ $C_1$—$C_6$-Alkyl; $C_7$—$C_{22}$-Alkyl; $C_1$—$C_6$-Alkylthio($C_1$—$C_6$)alkyl; $C_2$—$C_6$-Alkenylthio($C_1$—$C_6$)alkyl; Hydroxy($C_1$—$C_6$)alkyl; $C_1$—$C_6$-Alkanoyloxy($C_1$—$C_6$)alkyl; Phenyl($C_1$—$C_6$)alkoxy($C_1$—$C_6$)alkyl; Amino($C_1$—$C_6$)alkyl; $C_1$—$C_6$-Alkanoylamino($C_1$—$C_6$)alkyl; Benzamido($C_1$—$C_6$)alkyl; Phenyl($C_1$—$C_6$)alkoxycarbonylamino($C_1$—$C_6$)alkyl; Carboxy($C_1$—$C_6$)alkyl; $C_1$—$C_6$-Alkoxycarbonyl($C_1$—$C_6$)alkyl; Phenyl($C_1$—$C_6$)-alkoxycarbonyl($C_1$—$C_6$)alkyl; Phenylthio($C_1$—$C_6$)alkyl; Phenyl($C_1$—$C_6$)alkylthio($C_1$—$C_6$)alkyl; oder N—$C_1$—$C_6$-Alkylindolyl($C_1$—$C_6$)alkyl ist, und

$R^3$ und $R^4$ jeweils Wasserstoff oder $C_1$—$C_6$-Alkyl sind,

oder ein pharmazeutisch annehmbares Salz davon.

2. Eine Verbindung nach Anspruch 1, worin

$R^1$ Phenyl; Benzyl; Phenethyl; Diphenylmethyl; 1-Phenylethyl; 1-Naphthylmethyl; 2-Naphthylmethyl; 1-(1-Naphthyl)ethyl; 1-(2-Naphthyl)ethyl; 9-Anthrylmethyl; α-Methoxybenzyl; 4-Methoxybenzyl; 3-Indolylmethyl; Benzo[b]thien-3-yl; 2-Pyridylmethyl; 1-Methylindol-3-ylmethyl; 1-(1-Methylindol-3-yl)ethyl; oder 1-Methyl-2-phenylindol-3-ylmethyl ist;

$R^2$ Butyl; iso-Butyl; sec-Butyl; Pentyl; Octyl; Dodecyl; Methylthiomethyl; 2-Methylthioethyl; 3-Methylthiopropyl; Butylthiomethyl; Allylthiomethyl; Hydroxymethyl; Acetoxymethyl; Benzyloxymethyl; 3-Aminopropyl; 3-Acetamidopropyl; 3-Benzamidopropyl; 3-Benzyloxycarbonylaminopropyl; 2-Carboxyethyl; 2-Ethoxycarbonylethyl; 2-Benzyloxycarbonylethyl; Phenylthiomethyl; 2-Phenylthioethyl; Benzylthiomethyl; oder 1-Methylindol-3-ylmethyl ist; und

$R^3$ und $R^4$ jeweils Wasserstoff oder Methyl sind.

3. Eine Verbindung nach Anspruch 1, worin

$R^3$ und $R^4$ jeweils Wasserstoff oder $C_1$—$C_6$-Alkyl sind,

$R^1$ Mono(oder Di)phenyl($C_1$—$C_6$)alkyl; Naphthyl($C_1$—$C_6$)alkyl; Anthryl($C_1$—$C_6$)alkyl; N—$C_1$—$C_6$-Alkylindolyl($C_1$—$C_6$)alkyl; oder N—$C_1$—$C_6$-Alkylindolyl($C_1$—$C_6$)alkyl mit Phenyl am Indolring ist; und

$R^2$ $C_1$—$C_6$-Alkyl; $C_1$—$C_6$-Alkylthio($C_1$—$C_6$)alkyl; oder N—$C_1$—$C_6$-Alkylindolyl($C_1$—$C_6$)alkyl ist.

4. Eine Verbindung nach Anspruch 3, die (3R, 6R)-3-(2-Methylthioethyl)-6-[(R)-1-(1-naphthyl)-ethyl]piperazin-2,5-dion oder ein pharmazeutisch annehmbares Salz davon ist.

5. Verfahren zur Herstellung einer Piperazinverbindung der allgemeinen Formel

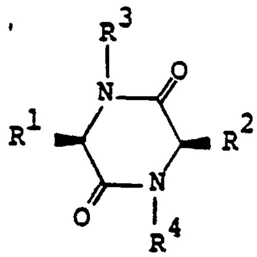

(Ia)

worin

$R^1$ Phenyl; Mono(oder Di)phenyl($C_1$—$C_6$)alkyl; Naphthyl($C_1$—$C_6$)alkyl; Anthryl($C_1$—$C_6$)alkyl; Phenyl($C_1$—$C_6$)alkyl mit $C_1$—$C_6$-Alkoxy Substituent(en); Indolyl($C_1$—$C_6$)alkyl; Benzothienyl($C_1$—$C_6$)alkyl; Pyridyl($C_1$—$C_6$)alkyl; N—$C_1$—$C_6$-Alkylindolyl($C_1$—$C_6$)alkyl; oder N—$C_1$—$C_6$-Alkylindolyl($C_1$—$C_6$)alkyl mit Phenyl-Substituent(en) am Indolring ist;

$R^2$ $C_1$—$C_6$-Alkyl; $C_7$—$C_{22}$-Alkyl; $C_1$—$C_6$-Alkylthio($C_1$—$C_6$)alkyl; $C_2$—$C_6$-Alkenylthio($C_1$—$C_6$)alkyl; Hydroxy($C_1$—$C_6$)alkyl; $C_1$—$C_6$-Alkanoyloxy($C_1$—$C_6$)alkyl; Phenyl($C_1$—$C_6$)alkoxy($C_1$—$C_6$)alkyl; Amino($C_1$—$C_6$)alkyl; $C_1$—$C_6$-Alkanoylamino($C_1$—$C_6$)alkyl; Benzamido($C_1$—$C_6$)alkyl; Phenyl($C_1$—$C_6$)alkoxycarbonylamino($C_1$—$C_6$)alkyl; Carboxy($C_1$—$C_6$)alkyl; $C_1$—$C_6$-Alkoxycarbonyl($C_1$—$C_6$)alkyl; Phenyl($C_1$—$C_6$)alkoxycarbonyl($C_1$—$C_6$)alkyl; Phenylthio($C_1$—$C_6$)alkyl; Phenyl($C_1$—$C_6$)alkylthio($C_1$—$C_6$)alkyl; oder N—$C_1$—$C_6$-Alkylindolyl($C_1$—$C_6$)alkyl ist, und

$R^3$ und $R^4$ jeweils Wasserstoff oder $C_1$—$C_6$-Alkyl sind,

oder eines Salzes davon, das umfaßt;

(a) Unterwerfen einer Verbindung der Formel

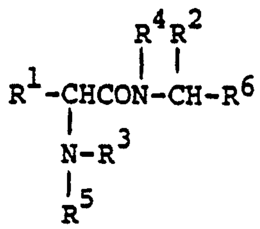

(II)

worin

$R^1$, $R^2$, $R^3$ und $R^4$ jeweils wie oben definiert sind,

$R^5$ Wasserstoff oder eine Aminoschutzgruppe ist, und

$R^6$ Carboxy oder geschütztes Carboxy ist,

oder deren reaktives Derivat an der Carboxy- oder Aminogruppe, oder eines Salzes davon einer Ringschlußreaktion, wobei man eine Verbindung der Formel

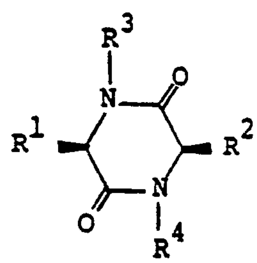

(Ia)

worin $R^1$, $R^2$, $R^3$ und $R^4$ jeweils wie oben definiert sind, oder ein Salz davon erhält;

(b) Umsetzen einer Verbindung der Formel

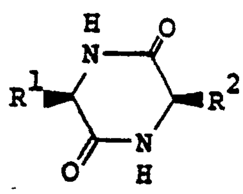

(Ib')

worin $R^1$ und $R^2$ jeweils wie oben definiert sind, oder eines Salzes davon, mit einem $C_1$—$C_6$-Alkylierungsmittel, wobei man eine Verbindung der Formel

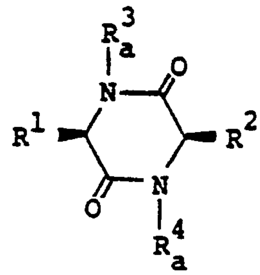

(Ic')

worin

$R^1$ und $R^2$ jeweils wie oben definiert sind, und

$R^3_a$ und $R^4_a$ jeweils $C_1$—$C_6$-Alkyl sind,

oder ein Salz davon erhält;

(c) Umsetzen einer Verbindung der Formel

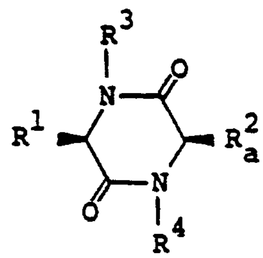

(Id')

worin

$R^1$, $R^3$ und $R^4$ jeweils wie oben definiert sind, und

$R^2_a$ Amino($C_1$—$C_6$)alkyl ist,

oder deren reaktiven Derivat an der Aminogruppe, oder eines Salzes davon, mit einem Acylierungsmittel, wobei man eine Verbindung der Formel

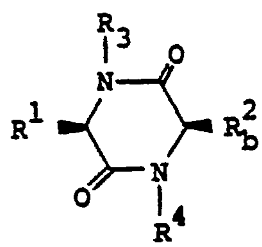

(Ie')

worin

$R^1$, $R^3$ und $R^4$ jeweils wie oben definiert sind, und

$R^2_b$ $C_1$—$C_6$-Alkanoylamino($C_1$—$C_6$)alkyl ist,

oder ein Salz davon erhält;

(d) Umsetzen einer Verbindung der Formel

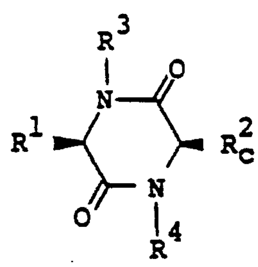

(If')

worin

$R^1$, $R^3$ und $R^4$ jeweils wie oben definiert sind, und

$R^2_c$ Hydroxy($C_1$—$C_6$)alkyl ist,

oder eines Salzes davon, mit einem Acylierungsmittel, wobei man eine Verbindung der Formel

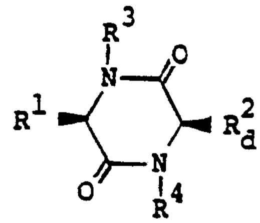

(Ig')

worin

$R^1$, $R^3$ und $R^4$ jeweils wie oben definiert sind, und

$R^2_d$ $C_1$—$C_6$ -Alkanoyloxy($C_1$—$C_6$)alkyl ist,

oder ein Salz davon erhält;

(e) Unterwerfen einer Verbindung der Formel

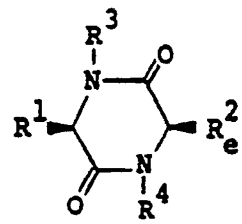

(Ih')

worin

$R^1$, $R^3$ und $R^4$ jeweils wie oben definiert sind, und

$R^2_e$ geschütztes Amino($C_1$—$C_6$)alkyl ist, oder eines Salzes davon,

einer Entfernungsreaktion der Aminoschutzgruppe, wobei man eine Verbindung der Formel

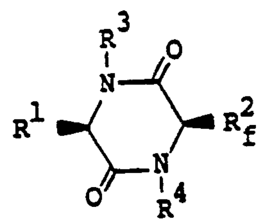

(Ii')

worin

$R^1$, $R^3$ und $R^4$ jeweils wie oben definiert sind, und

$R^2_f$ Amino($C_1$—$C_6$)alkyl ist,

oder ein Salz davon erhält; oder

(f) Unterwerfen einer Verbindung der Formel

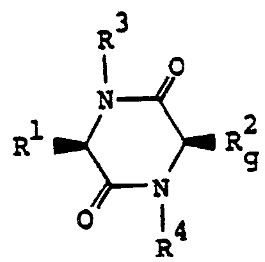

(Ij')

worin

$R^1$, $R^3$ und $R^4$ jeweils wie oben definiert sind, und

$R^2_g$ geschütztes Carboxy($C_1$—$C_6$)alkyl ist,

oder eines Salzes davon, einer Entfernungsreaktion der Carboxyschutzgruppe, wobei man eine Verbindung der Formel

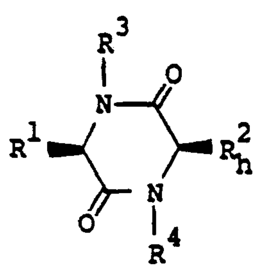

(Ik')

worin

$R^1$, $R^3$ und $R^4$ jeweils wie oben definiert sind, und

$R^2_h$ Carboxy($C_1$—$C_6$)alkyl ist,

oder ein Salz davon erhält.

6. Eine pharmazeutische Zusammensetzung, umfassend als einen wirksamen Inhaltsstoff, eine Piperazinverbindung der Formel:

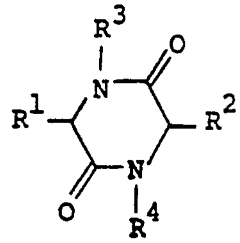

(I)

worin

$R^1$ Phenyl; Mono(oder Di)phenyl($C_1$—$C_6$)alkyl; Naphthyl($C_1$—$C_6$)alkyl; Anthryl($C_1$—$C_6$)alkyl; Phenyl($C_1$—$C_6$)alkyl mit $C_1$—$C_6$ Alkoxy-Substituent(en); Indolyl($C_1$—$C_6$)alkyl; Benzothienyl($C_1$—$C_6$)alkyl; Pyridyl($C_1$—$C_6$)alkyl; N—$C_1$—$C_6$-Alkylindolyl($C_1$—$C_6$)alkyl; oder N—$C_1$—$C_6$-Alkylindolyl($C_1$—$C_6$)alkyl mit Phenyl-Substituent(en) am Indolring ist;

$R^2$ $C_1$—$C_6$-Alkyl; $C_7$—$C_{22}$-Alkyl; $C_1$—$C_6$-Alkylthio($C_1$—$C_6$)alkyl; $C_2$—$C_6$-Alkenylthio($C_1$—$C_6$)alkyl; Hydroxy($C_1$—$C_6$)alkyl; $C_1$—$C_6$-Alkanoyloxy($C_1$—$C_6$)alkyl; Phenyl($C_1$—$C_6$)alkoxy($C_1$—$C_6$)alkyl; Amino($C_1$—$C_6$)alkyl; $C_1$—$C_6$-Alkanoylamino($C_1$—$C_6$)alkyl; Benzamido($C_1$—$C_6$)alkyl; Phenyl($C_1$—$C_6$)alkoxycarbonylamino($C_1$—$C_6$)alkyl; Carboxy($C_1$—$C_6$)alkyl; $C_1$—$C_6$-Alkoxycarbonyl($C_1$—$C_6$)alkyl; Phenyl($C_1$—$C_6$)-alkoxycarbonyl($C_1$—$C_6$)alkyl; Phenylthio($C_1$—$C_6$)alkyl; Phenyl($C_1$—$C_6$)alkylthio($C_1$—$C_6$)alkyl; N—$C_1$—$C_6$-Alkylindolyl($C_1$—$C_6$)alkyl ist, und

$R^3$ und $R^4$ jeweils Wasserstoff oder $C_1$—$C_6$-Alkyl sind,

und einen pharmazeutisch annehmbaren Träger.

7. Eine Piperazinverbindung der Formel

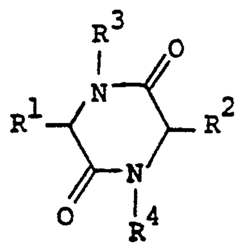

(I)

worin $R^1$, $R^2$, $R^3$ und $R^4$ jeweils wie in Anspruch 6 definiert sind, oder ein pharmazeutisch annehmbares Salz davon zur Verwendung als PAF-Antagonist.

8. Verfahren zur Herstellung einer pharmazeutischen Zusammensetzung, das umfaßt das Zusammenmischen einer Piperazinverbindung der Formel

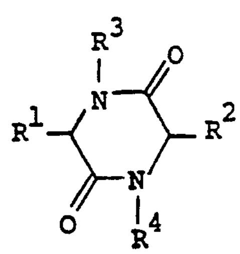

(I)

worin $R^1$, $R^2$, $R^3$ und $R^4$ jeweils wie in Anspruch 6 definiert sind, oder eines pharmazeutisch annehmbaren Salzes davon, und eines pharmazeutisch annehmbaren Trägers.

**Revendications**

1. Pipérazine répondant à la formule:

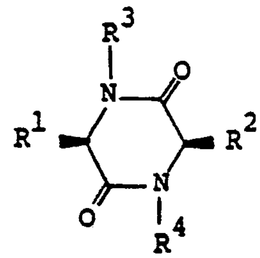

(Ia)

dans laquelle

$R^1$ est un groupe phényle; mono(ou di)phényl(alkyle en $C_1$ à $C_6$); naphtyl(alkyle en $C_1$ à $C_6$); anthryl(alkyle en $C_1$ à $C_6$); phényl(alkyle en $C_1$ à $C_6$) ayant un ou plusieurs substituants alcoxy en $C_1$ à $C_6$; indolyl(alkyle en $C_1$ à $C_6$); benzothiényl(alkyle en $C_1$ à $C_6$); pyridyl(alkyle en $C_1$ à $C_6$); N-(alkyle en $C_1$ à $C_6$)indolyl(alkyle en $C_1$ à $C_6$); ou N-(alkyle en $C_1$ à $C_6$)indolyl(alkyle en $C_1$ à $C_6$) ayant un ou plusieurs substituants phényle sur le noyau indole

$R^2$ est un groupe alkyle en $C_1$ à $C_6$; alkyle en $C_7$ à $C_{22}$; (alkyle en $C_1$ à $C_6$)thio(alkyle en $C_1$ à $C_6$); (alcényle en $C_2$ à $C_6$)thio(alkyle en $C_1$ à $C_6$); hydroxy(alkyle en $C_1$ à $C_6$); (alcanoyloxy en $C_1$ à $C_6$)(alkyle en $C_1$ à $C_6$); phényl(alcoxy en $C_1$ à $C_6$)(alkyle en $C_1$ à $C_6$); amino(alkyle en $C_1$ à $C_6$); (alcanoylamino en $C_1$ à $C_6$)(alkyle en $C_1$ à $C_6$); benzamido(alkyle en $C_1$ à $C_6$); phényl(alcoxy en $C_1$ à $C_6$)carbonylamino(alkyle en $C_1$ à $C_6$); carboxy(alkyle en $C_1$ à $C_6$); (alcoxy en $C_1$ à $C_6$)carbonyl(alkyle en $C_1$ à $C_6$); phényl(alcoxy en $C_1$ à $C_6$)carbonyl(alkyle en $C_1$ à $C_6$); phénylthio(alkyle en $C_1$ à $C_6$); phényl(alkylthio en $C_1$ à $C_6$)(alkyle en $C_1$ à $C_6$); ou N-(alkyle en $C_1$ à $C_6$)indolyl(alkyle en $C_1$ à $C_6$); et

$R^3$ et $R^4$ sont chacun un atome d'hydrogène ou un groupe alkyle en $C_1$ à $C_6$,

ou un de ses sels pharmaceutiquement acceptables.

2. Composé selon la revendication 1, dans lequel

$R^1$ est un groupe phényle; benzyle; phénéthyle; diphénylméthyle; 1-phényléthyle; 1-naphtylméthyle; 1-(1-naphtyl)éthyle; 1-(2-naphtyl)éthyle; 9-anthrylméthyle; α-méthoxybenzyle; 4-méthoxybenzyle; 3-indolylméthyle; benzo[b]thièn-3-yle; 2-pyridylméthyle; 1-méthylindol-3-ylméthyle; 1-(1-méthylindol-3-yl)éthyle; ou 1-méthyl-2-phénylindol-3-ylméthyle;

$R^2$ est un groupe butyle; isobutyle; sec-butyle; pentyle; octyle; dodécyle; méthylthiométhyle; 2-méthylthioéthyle; 3-méthylthiopropyle; butylthiométhyle; allylthiométhyle; hydroxyméthyle; acétoxyméthyle; benzyloxyméthyle; 3-aminopropyle; 3-acétamidopropyle; 3-benzamidopropyle; 3-benzyloxycarbonylaminopropyle; 2-carboxyéthyle; 2-éthoxycarbonyléthyle; 2-benzyloxycarbonyléthyle; phénylthiométhyle; 2-phénylthioéthyle; benzylthiométhyle; ou 1-méthylindol-3-ylméthyle; et

$R^3$ et $R^4$ sont chacun un atome d'hydrogène ou un groupe méthyle.

3. Composé selon la revendication 1, dans lequel

$R^3$ et $R^4$ sont chacun un atome d'hydrogène ou un groupe alkyle en $C_1$ à $C_6$;

$R^1$ est un groupe mono(ou di)phényl(alkyle en $C_1$ à $C_6$); naphtyl (alkyle en $C_1$ à $C_6$); anthryl(alkyle en $C_1$ à $C_6$); N-(alkyle en $C_1$ à $C_6$)indolyl(alkyle en $C_1$ à $C_6$); ou N-(alkyle en $C_1$ à $C_6$)indolyl(alkyle en $C_1$ à $C_6$) ayant un groupe phényle sur le noyau indole; et

$R^2$ est un groupe alkyle en $C_1$ à $C_6$; (alkylthio en $C_1$ à $C_6$)(alkyle en $C_1$ à $C_6$); ou N-(alkyle en $C_1$ à $C_6$)indolyle(alkyle en $C_1$ à $C_6$).

4. Composé selon la revendication 3, qui est la (3R, 6R)-3-(2-méthylthioéthyl)-6-[(R)-1-(1-naphtyl)éthyl]pipérazine-2,5-dione ou un de ses sels pharmaceutiquement acceptables.

5. Procédé pour préparer une pipérazine répondant à la formule:

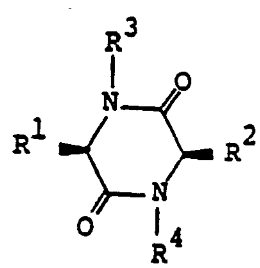

(Ia)

dans laquelle

$R^1$ est un groupe phényle; mono(ou di)phényl(alkyle en $C_1$ à $C_6$); naphtyl(alkyle en $C_1$ à $C_6$); anthryl(alkyle en $C_1$ à $C_6$); phényl(alkyle en $C_1$ à $C_6$) ayant un ou plusieurs substituants alcoxy en $C_1$ à $C_6$; indolyl(alkyle en $C_1$ à $C_6$); benzothiényl(alkyle en $C_1$ à $C_6$); pyridyl(alkyle en $C_1$ à $C_6$); N-(alkyle en $C_1$ à $C_6$)indolyl(alkyle en $C_1$ à $C_6$); ou N-(alkyle en $C_1$ à $C_6$)indolyl(alkyle en $C_1$ à $C_6$) ayant un ou plusieurs substituants phényle sur le noyau indole;

$R^2$ est un groupe alkyle en $C_1$ à $C_6$; alkyle en $C_7$ à $C_{22}$; (alkylthio en $C_1$ à $C_6$)(alkyle en $C_1$ à $C_6$); (alcénylthio en $C_2$ à $C_6$)(alkyle en $C_1$ à $C_6$); hydroxy(alkyle en $C_1$ à $C_6$); (alcanoyloxy en $C_1$ à $C_6$)(alkyle en $C_1$ à $C_6$); phényl(alcoxy en $C_1$ à $C_6$)(alkyle en $C_1$ à $C_6$); amino(alkyle en $C_1$ à $C_6$); (alcanoylamino en $C_1$ à $C_6$)(alkyle en $C_1$ à $C_6$); benzamido(alkyle en $C_1$ à $C_6$); phényl(alcoxy en $C_1$ à $C_6$)carbonylamino(alkyle en $C_1$ à $C_6$); carboxy(alkyle en $C_1$ à $C_6$); (alcoxy en $C_1$ à $C_6$)carbonyl(alkyle en $C_1$ à $C_6$); phényl(alcoxy en $C_1$ à $C_6$)carbonyl(alkyle en $C_1$ à $C_6$); phénylthio(alkyle en $C_1$ à $C_6$); phényl(alkylthio en $C_1$ à $C_6$)(alkyle en $C_1$ à $C_6$); ou N-(alkyle en $C_1$ à $C_6$)indolyl(alkyle en $C_1$ à $C_6$); et

$R^3$ et $R^4$ sont chacun un atome d'hydrogène ou un groupe alkyle en $C_1$ à $C_6$,

ou un de ses sels, qui comprend:

(a) le fait de soumettre un composé répondant à la formule:

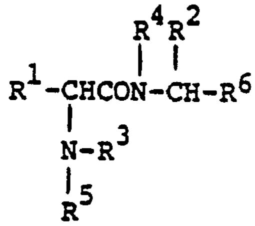

(II)

dans laquelle

$R^1$, $R^2$, $R^3$ et $R^4$ sont chacun tels que définis ci-dessus,

$R^5$ est un atome d'hydrogène ou un groupe protecteur du groupe amino, et

$R^6$ est un groupe carboxy ou carboxy protégé, ou son dérivé réacif sur le groupe carboxy ou amino, ou un de ses sels, à une réaction de fermeture de cycle, pour donner un composé répondant à la formule:

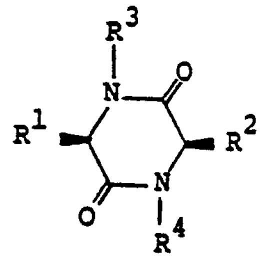

(Ia)

dans laquelle $R^1$, $R^2$, $R^3$ et $R^4$ sont chacun tels que définis ci-dessus, ou un de ses sels;

(b) le fait de faire réagir un composé répondant à la formule:

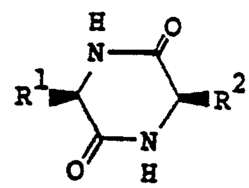

(Ib')

dans laquelle $R^1$ et $R^2$ sont chacun tels que définis ci-dessus, ou un de ses sels, avec un agent d'alkylation en $C_1$ à $C_6$, pour donner un composé répondant à la formule:

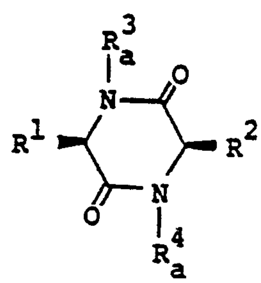

(Ic')

dans laquelle

$R^1$ et $R^2$ sont chacun tels que définis ci-dessus, et

$R^3_a$ et $R^4_a$ sont chacun un groupe alkyle en $C_1$ à $C_6$;

ou un de ses sels;

(c) le fait de faire réagir un composé répondant à la formule:

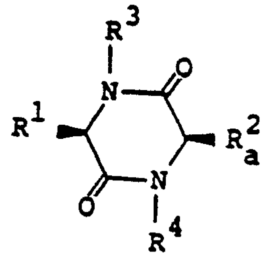

(Id')

dans laquelle

$R^1$, $R^3$ et $R^4$ sont chacun tels que définis ci-dessus, et

$R^2_a$ est un groupe amino(alkyle en $C_1$ à $C_6$),

ou son dérivé réactif sur le groupe amino, ou un de ses sels, avec un agent d'acylation, pour donner un composé répondant à la formule:

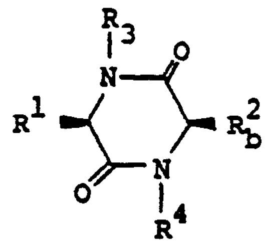

(Ie')

dans laquelle

$R^1$, $R^3$ et $R^4$ sont chacun tels que définis ci-dessus, et

$R^2_b$ est un groupe (alcanoylamino en $C_1$ à $C_6$)(alkyle en $C_1$ à $C_6$),

ou un de ses sels;

(d) le fait de faire réagir un composé répondant à la formule:

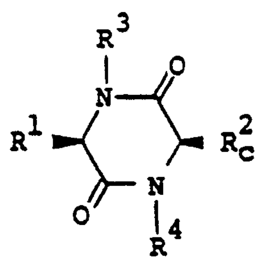

(If')

dans laquelle

$R^1$, $R^3$ et $R^4$ sont chacun tels que définis ci-dessus, et

$R^2_c$ est un groupe hydroxy(alkyle en $C_1$ à $C_6$),

ou un de ses sels, avec un agent d'acylation, pour donner un composé répondant à la formule:

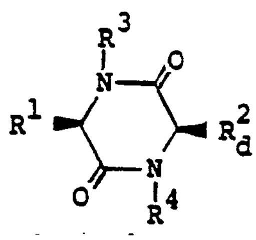

(Ig')

dans laquelle

$R^1$, $R^3$ et $R^4$ sont chacun tels que définis ci-dessus, et

$R^2_d$ est un groupe (alcanoyloxy en $C_1$ à $C_6$)(alkyle en $C_1$ à $C_6$);

ou un de ses sels;

(e) le fait de soumettre un composé répondant à la formule:

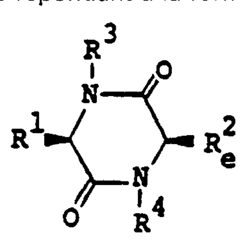

(Ih')

dans laquelle

$R^1$, $R^3$ et $R^4$ sont chacun tels que définis ci-dessus et

$R^2_e$ est un groupe amino(alkyle en $C_1$ à $C_6$)protégé,

ou un de ses sels, à une réaction d'élimination du groupe protecteur du groupe amino, pour donner un composé répondant à la formule:

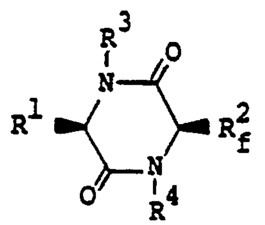

(Ii')

dans laquelle

$R^1$, $R^3$ et $R^4$ sont chacun tels que définis ci-dessus, et

$R^2_f$ est un groupe amino(alkyle en $C_1$ à $C_6$),

ou un de ses sels; ou (f) le fait de soumettre un composé répondant à la formule:

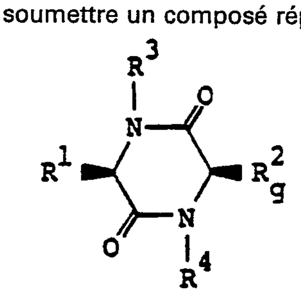

(Ij')

dans laquelle

$R^1$, $R^3$ et $R^4$ sont chacun tels que définis ci-dessus, et

$R^2_g$ est un groupe (carboxy protégé)(alkyle en $C_1$ à $C_6$),

ou un de ses sels, à une réaction d'élimination du groupe protecteur du groupe carboxy pour donner un composé répondant à la formule:

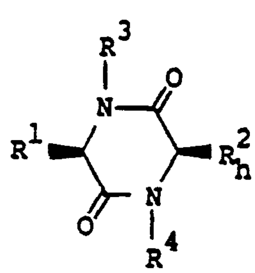

(Ik')

dans laquelle

$R^1$, $R^3$ et $R^4$ sont chacun tels que définis ci-dessus, et

$R^2_h$ est un groupe carboxy(alkyle en $C_1$ à $C_6$),

ou un de ses sels.

6. Composition pharmaceutique comprenant, comme ingrédient actif, une pipérazine répondant à la formule:

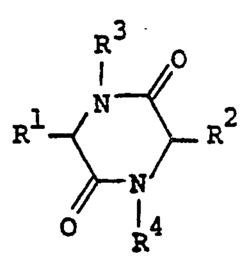

(I)

dans laquelle

$R^1$ est un groupe phényle; mono(ou di)phényl(alkyle en $C_1$ à $C_6$); naphtyl(alkyle en $C_1$ à $C_6$); anthryl(alkyle en $C_1$ à $C_6$); phényl(alkyle en $C_1$ à $C_6$) ayant un ou plusieurs substituants alcoxy en $C_1$ à $C_6$; indolyl(alkyle en $C_1$ à $C_6$); benzothiényl(alkyle en $C_1$ à $C_6$); pyridyl(alkyle en $C_1$ à $C_6$); N-(alkyle en $C_1$ à

$C_6$)indolyl(alkyle en $C_1$ à $C_6$); ou N-(alkyle en $C_1$ à $C_6$)indolyl(alkyle en $C_1$ à $C_6$) ayant un ou plusieurs substituants phényle sur le noyau indole;

$R^2$ est un groupe alkyle en $C_1$ à $C_6$; alkyle en $C_7$ à $C_{22}$; (alkylthio en $C_1$ à $C_6$)(alkyle en $C_1$ à $C_6$); (alcénylthio en $C_2$ à $C_6$)(alkyle en $C_1$ à $C_6$); hydroxy(alkyle en $C_1$ à $C_6$); (alcanoyloxy en $C_1$ à $C_6$)(alkyle en $C_1$ à $C_6$); phényl(alcoxy en $C_1$ à $C_6$)(alkyle en $C_1$ à $C_6$); amino(alkyle en $C_1$ à $C_6$); (alcanoylamino en $C_1$ à $C_6$)(alkyle en $C_1$ à $C_6$); benzamido(alkyle en $C_1$ à $C_6$); phényl(alcoxy en $C_1$ à $C_6$)carbonylamino(alkyle en $C_1$ à $C_6$); carboxy(alkyle en $C_1$ à $C_6$); (alcoxy en $C_1$ à $C_6$)carbonyl(alkyle en $C_1$ à $C_6$); phényl(alcoxy en $C_1$ à $C_6$)carbonyl(alkyle en $C_1$ à $C_6$); phénylthio(alkyle en $C_1$ à $C_6$); phényl(alkylthio en $C_1$ à $C_6$)(alkyle en $C_1$ à $C_6$) ou N-(alkyle en $C_1$ à $C_6$)indolyl(alkyle en $C_1$ à $C_6$); et

$R^3$ et $R^4$ sont chacun un atome d'hydrogène ou un groupe alkyle en $C_1$ à $C_6$,

ou un de ses sels pharmaceutiquement acceptables, et un support pharmaceutiquement acceptable.

7. Pipérazine répondant à la formule:

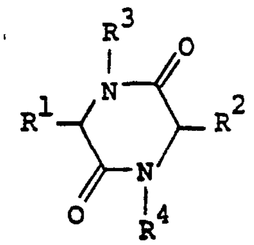

(I)

dans laquelle $R^1$, $R^2$, $R^3$ et $R^4$ sont chacun tels que définis dans la revendication 6, ou un de ses sels pharmaceutiquement acceptables pour l'utilisation comme antagoniste du FAP (facteur d'activation des plaquettes).

8. Procédé de préparation d'une composition pharmaceutique, qui comprend le mélange d'une pipérazine répondant à la formule:

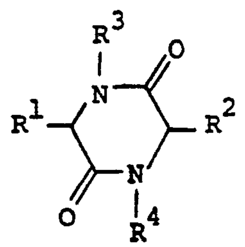

(I)

dans laquelle $R^1$, $R^2$, $R^3$ et $R^4$ sont chacun tels que définis dans la revendication 6, ou d'un de ses sels pharmaceutiquement acceptables, et d'un support pharmaceutiquement acceptable.